(19) Europäisches Patentamt
European Patent Office
Office européen des brevets

(11) EP 3 047 845 B1

(12) **EUROPÄISCHE PATENTSCHRIFT**

(45) Veröffentlichungstag und Bekanntmachung des Hinweises auf die Patenterteilung:
**28.06.2017 Patentblatt 2017/26**

(51) Int Cl.:
***A61K 8/895*** (2006.01) ***C08L 83/04*** (2006.01)

(21) Anmeldenummer: **15152458.4**

(22) Anmeldetag: **26.01.2015**

(54) **Silikongele für insbesondere kosmetische Anwendungen**

Silicone gels in particular for cosmetic applications

Gels de silicone pour des applications plus particulièrement cosmétiques

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**27.07.2016 Patentblatt 2016/30**

(73) Patentinhaber: **Evonik Degussa GmbH
45128 Essen (DE)**

(72) Erfinder:
- **Klostermann, Dr. Michael
45239 Essen (DE)**
- **Wiechers, Dr. Susann
45239 Essen (DE)**
- **Meyer, Dr. Jürgen
45134 Essen (DE)**
- **Venzmer, Dr. Joachim
45239 Essen (DE)**
- **Henning, Dr. Frauke
45259 Essen (DE)**
- **Mentel, Dr. Matthias
44357 Dortmund (DE)**

(56) Entgegenhaltungen:
**WO-A2-2011/049896**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 047 845 B1

Printed by Jouve, 75001 PARIS (FR)

## Beschreibung

### Gebiet der Erfindung

Gegenstand der Erfindung ist ein Verfahren zur Herstellung von Silikongelen sowie deren Verwendung in insbesondere kosmetischen Formulierungen.

### Stand der Technik

Ein wichtiges Merkmal von kosmetischen Formulierungen ist oftmals, dass sie nach Applikation auf der Haut zu einem samtig-seidigen Hautgefühl führen. Es ist hinlänglich bekannt, dass sich diese Eigenschaften durch die Verwendung von niedermolekularen Silikonen wie z.B. niedrigviskose Silikonöle erzielen lassen. Ein Nachteil dieser Substanzklasse ist jedoch, dass sich bedingt durch ihre gute Fließfähigkeit das von ihnen erzeugte seidige Hautgefühl nach der Applikation relativ schnell verflüchtigt. Zur Lösung dieses Problems wurden in der Vergangenheit eine Reihe von vernetzten, polymeren Silikongel-Materialien entwickelt, welche sich durch ein deutlich länger anhaltendes samtig-seidiges Hautgefühl auszeichnen. Diese Silikongel-Materialien enthalten vernetze Silikonpolymere, welche in einem kosmetisch verträglichen Lösemittel hergestellt wurden. Die Verwendung solcher vernetzen Silikongele in kosmetischen Formulierungen ist beispielsweise in den Schriften US5760116 bzw. US6936686 beschrieben. Im Stand der Technik werden solche vernetzten Silikongele meist durch Hydrosililierungsreaktionen eines Si-H-funktionellen Siloxans mit einem olefinisch modifizierten Siloxan in Gegenwart eines Platinkatalysators hergestellt.

Die Schriften WO12065822, US20030049212 sowie US2004228821 offenbaren überdies Silikongele, welche in Gegenwart eines Peroxo-Initiators aus thermisch vernetzbaren Silikonen hergestellt werden können. Strukturmerkmale der zur Herstellung dieser Gele eingesetzten radikalisch polymerisierbaren Silikonmakromonomere werden in diesen Schriften jedoch nicht offenbart.

In der Schrift WO2011049896 wird die Herstellung und Verwendung von radikalisch vernetzten Silikongelen beschrieben, welche in Gegenwart eines Organoboran-Initiators polymerisiert wurden. Als Trägermedium werden sowohl Silikonöle wie Decamethylcyclopentasiloxan als auch organische Öle, z.B. kosmetische Ester aufgeführt. Als Makromonomere werden in dieser Schrift eine breite, sehr unspezifische Auswahl an radikalisch polymerisierbaren Siloxanen beschrieben, deren Einfluss auf die sensorischen Eigenschaften der hieraus resultierenden Silikongele wird jedoch nicht dargestellt.

Aus dem Stand der Technik ist daher nicht ersichtlich, welche strukturellen Eigenschaften bei der Auswahl eines radikalisch polymerisierbaren Silikonmakromonomers berücksichtigt werden müssen, um zu Silikongele mit bevorzugten haptischen Eigenschaften, wie z.B. einem besonders samtig-seidigen Hautgefühl, zu gelangen.

Die in der WO2011049896 beschriebenen Silikongele haben überdies den Nachteil, dass die als Radikalstarter eingesetzten Boraninitiatoren, meist Trialkylborane, ein akute Ätzwirkung auf Augen und Haut aufweisen und so bei Exposition zu schweren Augen und Hautschäden führen können. Kurzkettige Alkylborane, wie z.B. Triethylboran, weisen zudem eine akute orale Toxizität auf. In Folge dessen ist die Verwendung von Boran-initüerten Silikongelen in kosmetischen Anwendungen kritisch. Die durch Solvolyse aus Alkylboranen entstehenden Borate wurden außerdem als reproduktionstoxisch eingestuft und werden in der Kosmetik reguliert [SCCS (Scientific Committee on Consumer Safety), Opinion on boron compounds, 22 June 2010]. Neben gesundheitsschädlichen Aspekten haben Alkylborane zudem den Nachteil, dass es sich bei dieser Substanzklasse um pyrophore Verbindungen handelt, was ihr Handhabung während der Herstellung der Silikongele erschwert. Ein weiterer Nachteil dieser Boraninitiatoren ist, dass sie oft nur dann eine ausreichend Reaktivität aufweisen, wenn sie in Form eines Boran-Amin-Komplexes vorliegen. Die Verwendung von Aminverbindungen in kosmetischen Anwendungen ist jedoch ebenfalls unerwünscht, insbesondere im Hinblick auf olfaktorische Einflüsse solcher Substanzen. Eine weiterer Nachteil vieler im Stand der Technik beschriebenen Silikongel-Materialien ist zudem, dass bei der Verwendung eines organischen Trägermediums das Silikonpolymer oftmals nur eine unzureichende Kompatibilität zum Trägermedium aufweist. Hierdurch kann es zu einer Verklumpung des Silikonpolymers im Gel bis hin zu einer makroskopischen Phasenseparation führen, was negative Auswirkungen auf das Hautgefühl entsprechender Materialien zur Folge hat. Zudem haben viele gängige Silikongel-Materialien den Nachteil, dass sie sich durch eine sehr hohe Viskosität sowie durch schlechte Fließeigenschaften auszeichnen. Dies ist sowohl bei der Herstellung als auch bei der Verarbeitung dieser Materialien nachteilig, da deren Handhabbarkeit durch die fehlende Pumpbarkeit stark eingeschränkt ist.

Aufgabe der Erfindung war es daher, radikalisch vernetzte Silikongele bereitzustellen, welche sich durch ein langanhaltendes, samtiges Hautgefühl sowie durch eine gute Verarbeitbarkeit auszeichnen.

### Beschreibung der Erfindung

Überraschenderweise wurde gefunden, dass die der Erfindung gestellte Aufgabe gelöst wird durch Silikongele

erhältlich durch die radikalische Polymerisation eines Silikons enthaltend mindestens einen Rest ausgewählt aus Acrylat und Methacrylat in einem kosmetisch geeigneten Trägermedium, unter der Maßgabe, dass das mittlere molare Verhältnis von Acrylat- und Methacrylat-Resten, gegebenenfalls in Summe, zu Si Atomen im Silikon im Bereich von 0,014 bis 0,15 liegt.

Gegenstand der vorliegenden Erfindung ist daher ein Verfahren zur Herstellung von Silikongelen umfassend die Verfahrensschritte

A) Bereitstellen einer Mischung umfassend
ein Silikon enthaltend
mindestens eine (Meth-)Acrylat-Gruppe der Formel (I)

$$-O-C(O)-CR=CH_2 \qquad \text{Formel (I)}$$

mit R = -H oder $-CH_3$,
mit der Maßgabe, dass das mittlere molare Verhältnis von (Meth-)Acrylat-Gruppen zu Si Atomen im Silikon in einem Bereich von 0,014 - 0,15 liegt, und
mindestens ein kosmetisch geeignetes Trägermedium, insbesondere ein kosmetisches Öl,
B) Initiieren einer radikalischen Polymerisation durch Zugabe mindestens eines organischen Radikalstarters, bevorzugt eines organischen Peroxids.

Ein weiterer Gegenstand der Erfindung sind die mit dem erfindungsgemäßen Verfahren herstellbaren Silikongele sowie kosmetische Formulierungen enthaltend diese.

Ein Vorteil der vorliegenden Erfindung ist, dass sich die erfindungsgemäßen Silikongele bei Applikation auf der Haut durch ein samtig-seidiges Hautgefühl auszeichnen. Verwendet als Additiv in kosmetischen Formulierungen wie Cremes und Lotionen führen die Silikongele zudem zu einem reduzierten wachsig / öligen Hautgefühl. Diese sensorischen Vorteile sind hierbei bereits bei niedrigen Silikonpolymer-Konzentrationen deutlich bemerkbar, wobei die Wirkung der Silikongele auf der Haut auch noch nach längerer Zeit nach Applikation fühlbar ist. Im Vergleich zu diesen erfindungsgemäßen Silikongelen wurde gefunden, dass sich nichterfindungsgemäße Silikongele, welche auf Silikon(meth)acrlyaten dadurch gekennzeichnet, dass das mittlere molare Verhältnis von (Meth-)Acrylat-Gruppen zu Si Atomen größer 0,15 ist, basieren, durch ein stumpfes, wenig seidiges Hautgefühl auszeichnen. Im Falle von Silikongelen basierend auf Silikon(meth)acrylaten mit einem mittleren molaren Verhältnis von (Meth-)Acrylat-Gruppen zu Si Atomen kleiner 0,014 wurde festgestellt, dass solche nichterfindungsgemäßen Gele zu einem sehr öligen / fettigen sensorischen Eindruck auf der Haut führen, was ebenfalls unerwünscht ist.

Bedingt durch ihre noch relative gute Fließfähigkeit ist ein weiterer Vorteil der erfindungsgemäßen Silikongele ihre gute Handhabbarkeit sowie Verarbeitbarkeit.

In einer bevorzugten Ausführungsform des erfindungsgemäßen Verfahrens werden Silikone eingesetzt, die mindestens eine Alkylseitenketten mit 2 bis 30, bevorzugt 8 bis 24, besonders bevorzugt 12 bis 18, C-Atomen aufweisen. Dies hat eine erhöhte Kompatibilität der erhaltenen Silikonpolymere mit organischen Trägermedien, z.B. mit kosmetischen Estern zur Folge, was ebenfalls zu einem verbesserten Hautgefühl entsprechender Silikongele führt.

Das beanspruchte Verfahren zur Herstellung von Silikongelen umfasst die Verfahrensschritte

A) Bereitstellen einer Mischung umfassend
ein Silikon enthaltend mindestens eine (Meth-)Acrylat-Gruppe der Formel (I)

$$-O-C(O)-CR=CH_2 \qquad \text{Formel (I)}$$

mit R = -H oder $-CH_3$,
mit der Maßgabe, dass das mittlere molare Verhältnis von (Meth-)Acrylat-Gruppen zu Si Atomen im Silikon in einem Bereich von 0,014 - 0,15 liegt, und mindestens ein kosmetisches Öl,
B) Initiieren einer radikalischen Polymerisation durch Zugabe mindestens eines organischen Radikalstarters, bevorzugt eines organischen Peroxids.

Unter dem Begriff "Silikon" im Zusammenhang mit der vorliegenden Erfindung sind Verbindungen aus der Klasse der Poly-Organosiloxane zu verstehen.

Unter dem Begriff "Silikongel" im Zusammenhang mit der vorliegenden Erfindung ist ein makroskopisch homogenes System, welches auf mikroskopischer Ebene mindestens ein vernetztes Silikon-Polymer enthält, dessen Netzwerkstruktur durch eine zweite flüssige Phase gequollen ist, zu verstehen.

Unter dem Begriff "kosmetisches Öl" im Zusammenhang mit der vorliegenden Erfindung sind mit Wasser nicht-mischbare Flüssigkeiten, welche zur Herstellung kosmetischer Formulierungen geeignet sind, zu verstehen. Mit Wasser nicht mischbar bedeutet im Zusammenhang mit der vorliegenden Erfindung, dass bei Raumtemperatur wässrige Mischungen von kosmetischen Ölen bei Ölkonzentrationen von 0,5 - 99,5 vol.-% bezogen auf die Gesamtmischung zu einer bereits mit dem menschlichen Auge wahrnehmbaren Trübung bzw. zur Ausbildung von zwei oder mehr Phasen führen. Des Weiteren sind im Zusammenhang mit der vorliegenden Erfindung kosmetische Öle bevorzugt dadurch gekennzeichnet, dass sie zu Wasser ein Grenzflächenspannung von > 5 mN/m aufweisen. Kosmetische Öle können zum Beispiel Oleochemie- oder Silikonchemie basiert sein.

Unter dem Begriff "organischer Radikalstarter" im Zusammenhang mit der vorliegenden Erfindung sind organische Verbindungen zu verstehen, die mindestens eine labile, homolytisch spaltbare Bindung, wie Beispielsweise eine O-O- oder eine N=N-Bindung, aufweisen und so bei zum Beispiel Temperaturerhöhung in Radikale zerfallen können.

Unter dem Begriff "langkettig" im Zusammenhang mit der vorliegenden Erfindung ist eine Verbindung, die mindestens 8, insbesondere 8 bis 30, Kohlenstoffatome aufweist, zu verstehen.

Alle angegebenen Prozent (%) sind, wenn nicht anders angegeben, Massenprozent.

In einem bevorzugten erfindungsgemäßen Verfahren beträgt die Konzentration des Silikons in der Silikon-Öl-Mischung von 1 bis 40 Gew.-%, bevorzugt von 3 bis 30 Gew.-%, besonders bevorzugt von 5 bis 25 Gew.-%, bezogen auf das Gesamtgewicht der Silikon-Öl-Mischung.

Bevorzugte erfindungsgemäße Verfahrene sind dadurch gekennzeichnet, dass das mittlere molare Verhältnis von (Meth-)Acrylat-Gruppen zu Si Atomen im Silikon im Bereich von 0,016 bis 0,12, besondere bevorzugt von 0,018 bis 0,10, insbesondere bevorzugt von 0,020 bis 0,075, liegt.

In dem erfindungsgemäßen Verfahren werden bevorzugt Silikone eingesetzt, die verzweigte oder unverzweigte Alkylreste aufweisen, welche sich von langkettigen Fettsäuren, wie z.B. Capryl-Lauryl-, Myrstyl-, Cetyl-, Stearyl-, oder Behenylreste, ableiten. Ganz besonders bevorzugt werden mit Cetyl-Resten substituierte Silikone eingesetzt

Besonders bevorzugt werden im erfindungsgemäßen Verfahren Silikone der allgemeinen Formel II) eingesetzt

$$M_a M'_{a1} M''_{a2} D_b D'_{b1} D''_{b2} T_c T'_{c1} T''_{c2} Q_d \qquad \text{Formel (II)},$$

wobei

M = $(R^1_3\ Si\ O_{1/2})$
M' = $(R^2 R^1_2\ Si\ O_{1/2})$
M" = $(R^3 R^1_2\ Si\ O_{1/2})$
D = $(R^1_2\ Si\ O_{2/2})$
D' = $(R^2 R^1\ Si\ O_{2/2})$
D" = $(R^3 R^1\ Si\ O_{2/2})$
T = $(R^5\ Si\ O_{3/2})$
T' = $(R^2\ Si\ O_{3/2})$
T" = $(R^3\ Si\ O_{3/2})$
Q = $(Si\ O_{4/2})$

a = 0 bis 32; bevorzugt 0 bis 22, insbesondere >= 2;
a1 = 0 bis 32, bevorzugt 0 bis 22, insbesondere 0;
a2 = 0 bis 32; bevorzugt 0 bis 22, insbesondere >= 2;

mit der Maßgabe, dass

$$a + a1 + a2 >= 2,$$

bevorzugt = 2;

b = 1 bis 600, bevorzugt 10 bis 500, insbesondere 20 bis 400;
b1 = 0 bis 80, bevorzugt 0 bis 50, insbesondere 1 bis 20;
b2 = 0 bis 50, bevorzugt 0 bis 20, insbesondere 0 bis 10;

c = 0 bis 30, bevorzugt 0 bis 20, insbesondere 0;
c1 = 0 bis 10, bevorzugt 0 bis 5, insbesondere 0;

c2 = 0 bis 10, bevorzugt 0 bis 5, insbesondere 0;

d = 0 bis 15, bevorzugt 0 bis 12, insbesondere 0;

mit der Maßgabe, dass

$$a2 + b2 + c2 >= 1$$

und

$R^1$ = unabhängig voneinander gleiche oder verschiedene lineare oder verzweigte, gegebenenfalls aromatische Kohlenwasserstoffreste mit 1 bis 30 Kohlenstoffatomen, bevorzugt mit 1 bis 6 Kohlenstoffatomen, besonders bevorzugt Methyl oder Phenyl, insbesondere Methyl;
$R^2$ = unabhängig voneinander gleiche oder verschiedene lineare oder verzweigte, gegebenenfalls aromatische Kohlenwasserstoffreste mit 2 bis 30, bevorzugt 7 bis 22, Kohlenstoffatomen, bevorzugt lineare gesättigte Kohlenwasserstoffreste mit 6 bis 30, bevorzugt 7 bis 22, Kohlenstoffatomen, besonders bevorzugt lineare gesättigte Kohlenwasserstoffreste mit 8 bis 22, insbesondere 12 bis 18 Kohlenstoffatomen, insbesondere Dodecyl-, Tetradecyl-, Hexadecyl- und Octadecylreste;
$R^3$ = unabhängig voneinander gleiche oder verschiedene organische Reste, die eine (Meth-)Acrylat-Gruppe der Formel (I)

$-O-C(O)-CR=CH_2$ Formel (I)

mit R = -H oder $-CH_3$, aufweisen;
$R^5$ = unabhängig voneinander gleiche oder verschiedene Reste $R^1$, $R^2$ oder $R^3$, bevorzugt $R^1$, insbesondere Methyl, Phenyl, Dodecyl oder Hexadecyl,
sind.

Die Werte a, a2, a3, b, b1, b2, c, c1, c2 und d stellen hierbei statistische Mittelwerte da.

In einer bevorzugten Ausführungsform der Erfindung werden Silikone gemäß Formel (II) eingesetzt, für die gilt

$$a + a1 + a2 = 2$$

und

$$c + c1 + c2 + d = 0.$$

In einer weiteren bevorzugten Ausführungsform der Erfindung werden Silikone gemäß Formel (II) eingesetzt für die gilt

$$a2 = 2$$

und

$$c + c1 + c2 + d = 0.$$

Besonders bevorzugte in dem erfindungsgemäßen Verfahren eingesetzte Silikone weisen Reste, die mindestens eine (Meth-)Acrylat-Gruppe der Formel (I) enthalten, auf, ausgewählt aus der Gruppe

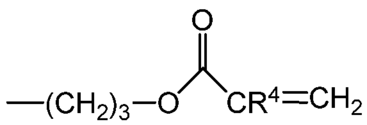

(Ia),

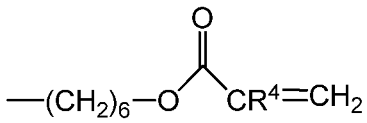

(Ib),

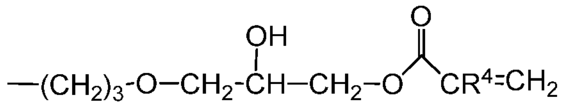

(Ic),

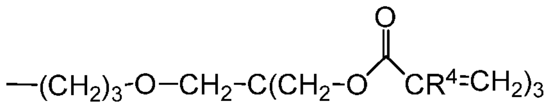

(Id),

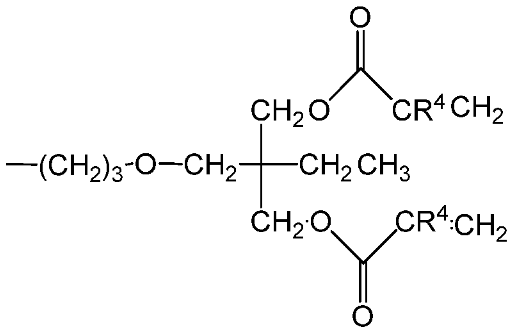

(Ie),

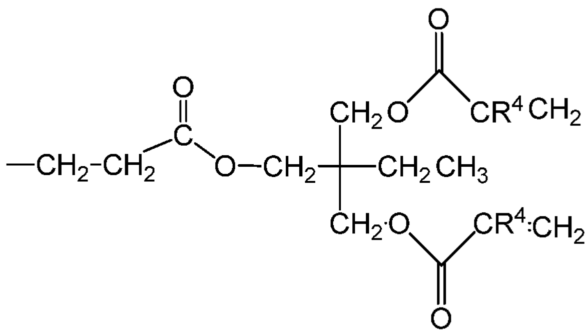

(If),

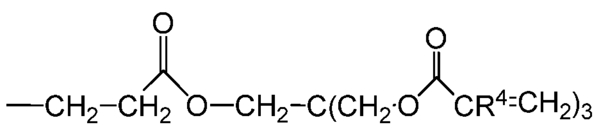

(Ig),

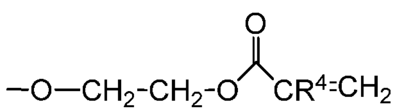

(Ih),

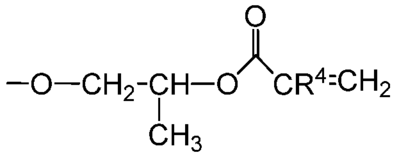

(Ii)

und

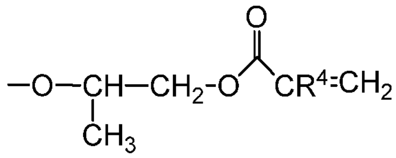

(Ij),

wobei $R^4$ Wasserstoff oder eine Methylgruppe, vorzugsweise eine Methylgruppe, ist. Ganz besonders bevorzugt eingesetzte Silikone, die mindestens eine (Meth-)Acrylat-Gruppe der Formel (I) aufweisen, sind solche, die diese in Form eines Restes ausgewählt aus der Gruppe umfassend die Reste mit den Formeln (Ib) und (Ie) aufweisen, wobei $R^4$ eine Methylgruppe ist.

In einer besonders bevorzugten Ausführungsform der Erfindung werden Silikone gemäß Formel (II) eingesetzt, für die gilt:

a2=2,
b = 20 bis 400,
b2 = 1 bis 20
$R^1 = CH_3$,
$R^3 = -C_6H_{12}-O-C(O)-C(CH_3)=CH_2$,
sowiea=a1 =b1 =c=c1 =c2=d=0;
a2=2,
b = 20 bis 400,
$R^1 = CH_3$,
$R^3 = (CH_2)_3-O-CH_2-C(CH_2-O-C(O)-C(CH_3)=CH_2)_2-CH_2-CH_3$,
sowiea=a1 =b1 =b2=c=c1 =c2=d=0;
a2=2,
b = 20 bis 400,
b1 = 1 bis 20,
$R^1 = CH_3$,
$R^2$ = C6 bis C24 n-Alkyl,
$R^3 = (CH_2)_3-O-CH_2-C(CH_2-O-C(O)-C(CH_3)=CH_2)_2-CH_2-CH_3$,
sowie
a=a1 =b2=c=c1 =c2=d=O;oder
a + a2 =4,
b = $_{20}$ bis $_{400}$,
b1 = 1 bis 20,
c = 2,
$R^1 = R^5 = CH_3$,
$R^2$ = C6 bis C24 n-Alkyl,
$R^3 = (CH_2)_3-O-CH_2-C(CH_2-O-C(O)-C(CH_3)=CH_2)_2-CH_2-CH_3$,
sowie a1 = b2 = c1 = c2 = d =0

Im erfindungsgemäßen Verfahren können die Silikone enthaltend mindestens eine (Meth-)Acrylat-Gruppe der Formel (I), insbesondere die der Formel (II) einzeln oder als Mischungen, insbesondere als statistische Mischungen, eingesetzt werden. Vorzugsweise werden in dem erfindungsgemäßen Verfahren Mischungen von Silikonen, insbesondere solche der Formel (II) eingesetzt, in denen die Silikone sich bezüglich ihrer Struktur und/oder ihrem Molekulargewicht unterscheiden.

Erfindungsgemäß bevorzugt werden in dem erfindungsgemäßen Verfahren kosmetische Öle als kosmetisch geeignetes Trägermedium eingesetzt, insbesondere ausgewählt aus der Gruppe der Fettalkohole, Ester von linearen

Fettsäuren mit linearen oder verzweigten Fettalkoholen, Ester von verzweigten Fettsäuren mit linearen oder verzweigten Fettalkoholen, Ester von linearen Fettsäuren mit unverzweigten oder verzweigten mehrwertigen Alkoholen, Ester von verzweigten Fettsäuren mit unverzweigten oder verzweigten mehrwertigen Alkoholen, Ester von linearen Fettsäuren mit unverzweigten oder verzweigten Alkoholen, Ester von verzweigten Fettsäuren mit unverzweigten oder verzweigten Alkoholen, Ester von Alkylhydroxycarbonsäuren mit linearen oder verzweigten Fettalkoholen. Des Weiteren Mono-, Di- oder Triglyceride in flüssiger oder fester Form. Des Weiteren Ester von Carbonsäuren, aromatischen Carbonsäuren oder Dicarbonsäuren mit linearen oder verzweigten Fettalkoholen, unverzweigten oder verzweigten mehrwertigen Alkoholen oder unverzweigten oder verzweigten Alkoholen. Des Weiteren lineare, cyclische oder verzweigte Kohlenwasserstoffe, mit oder ohne Substituenten, mit oder ohne Doppelbindungen. Des Weiteren pflanzliche Öle, Carbonate mit unverzweigten oder verzweigten Alkoholen, Carbonate mit unverzweigten oder verzweigten mehrwertigen Alkoholen, Carbonate mit linearen oder verzweigten Fettalkoholen. Des Weiteren Ether, mit oder ohne Alkoxygruppen, Silikonöle, mit oder ohne organische Modifizierung. Des Weiteren Mischungen dieser Öle in beliebigen Verhältnissen., bevorzugt Ester von linearen Fettsäuren mit linearen oder verzweigten Fettalkoholen, Ester von verzweigten Fettsäuren mit linearen oder verzweigten Fettalkoholen, Ester von linearen Fettsäuren mit unverzweigten oder verzweigten mehrwertigen Alkoholen, Ester von verzweigten Fettsäuren mit unverzweigten oder verzweigten mehrwertigen Alkoholen, Ester von linearen Fettsäuren mit unverzweigten oder verzweigten Alkoholen, Ester von verzweigten Fettsäuren mit unverzweigten oder verzweigten Alkoholen. Des Weiteren Mono-, Di- oder Triglyceride in flüssiger oder fester Form. Des Weiteren Ester von Carbonsäuren, aromatischen Carbonsäuren oder Dicarbonsäuren mit linearen oder verzweigten Fettalkoholen, unverzweigten oder verzweigten mehrwertigen Alkoholen oder unverzweigten oder verzweigten Alkoholen. Des Weiteren lineare, cyclische oder verzweigte Kohlenwasserstoffe, mit oder ohne Substituenten, mit oder ohne Doppelbindungen. Des Weiteren pflanzliche Öle, Carbonate mit unverzweigten oder verzweigten Alkoholen, Carbonate mit unverzweigten oder verzweigten mehrwertigen Alkoholen, Carbonate mit linearen oder verzweigten Fettalkoholen., besonders bevorzugt lineare, cyclische oder verzweigte Kohlenwasserstoffe, mit oder ohne Substituenten, mit oder ohne Doppelbindungen. Des Weiteren Carbonate mit unverzweigten oder verzweigten Alkoholen, Carbonate mit unverzweigten oder verzweigten mehrwertigen Alkoholen, Carbonate mit linearen oder verzweigten Fettalkoholen. Ester von verzweigten Fettsäuren mit unverzweigten oder verzweigten mehrwertigen Alkoholen

Verfahrensschritt B) des erfindungsgemäßen Verfahrens entspricht im Wesentlichen einer herkömmlichen radikalischen Polymerisation, welche durch ein Peroxid initüert wird. Entsprechende radikalische Polymerisationen sind beispielsweise in B. Tieke, "Makromolekulare Chemie: Eine Beinführung‘; 2005, Wiley-VCH Verlag, Deutschland beschrieben.

In einem bevorzugten erfindungsgemäßen Verfahren ist der organische Radikalstarter ein organisches Peroxid, besonders bevorzugt ausgewählt aus der Gruppe der Substanzen, die nach dem Radikalstart zu Verbindungen werden, die in kosmetischen Produkten verträglich sind, wie z. B. Kohlenwasserstoffe, Alkohole oder Säuren mit 6 bis 30 Kohlenstoffatomen. Als Radikalstarter können insbesondere Peroxide eingesetzt werden, die sich von organischen Säuren, insbesondere Fettsäuren ableiten. Unter diesen, auch als Diacylperoxide bekannten Verbindungen, sind Dilauroylperoxid, Didecanoylperoxid und Düsononanoylperoxid bevorzugt. Besonders bevorzugt wird als Radikalstarter Dilauroylperoxid eingesetzt. Die Initiierung erfolgt vorzugsweise durch Temperaturerhöhung.

Ein Vorteil ist, dass solche Radikalstarter sowie deren Zerfallsprodukte in der Regel gesundheitlich unbedenklich sind, wodurch die Silikongele bedenkenlos in kosmetischen Anwendungen eingesetzt werden können. Insbesondere bei einer Initiierung durch Dilaurolyperoxid wird im Verlauf der Polymerisation als einziges Zerfallsprodukt Laurinsäure erhalten, welche ein gängiger Rohstoff in kosmetischen Anwendungen ist.

Der Einsatz von organischen Radikalstarten in dem erfindungsgemäßen Verfahren hat zudem den Vorteil, dass sie eine ausreichende Aktivität auch ohne den Zusatz eines Wirksamkeitsverstärkers aufweisen, wie es beispielsweise bei der Verwendung von Boraninitiatoren erforderlich wäre. Dies bietet bei der Herstellung der Gele sowohl prozessökologische wie auch prozessökonomische Vorteile.

In einem weiteren erfindungsmäßig bevorzugten Verfahren wird das organische Peroxid in einer Konzentration von 0,01 bis 5 Gew.-%, bevorzugt von 0,05 bis 2 Gew.-%, besonders bevorzugt zwischen von 0,1 bis 1 Gew.-%, bezogen auf die Gesamtansatzgröße eingesetzt.

Die Silikongele, die nach dem erfindungsgemäßen Verfahren erhältlich sind, zeichnen sich durch die Maßgabe aus, dass sie ein Silikon(meth)acrylat-Polymernetzwerk, gequollen mit einem kosmetischen Öl, aufweisen, welches aus einem Silikon(meth)acrylat, welches dadurch gekennzeichnet ist, dass das mittlere molare Verhältnis von (Meth-)Acrylat-Gruppen zu Si Atomen im Bereich von 0,014 bis 0,15, insbesondere von 0,016 bis 0,12, besondere bevorzugt von 0,018 bis 0,10, insbesondere bevorzugt von 0,020 bis 0,075, liegt, hervorgeht. Das Verhältnis von (Meth-)Acrylat-Gruppen zu Si Atomen bestimmt hierbei die Dichte an theoretisch möglichen Netzwerkpunkten im Polymernetzwerk. Durch das erfindungsgemäße Verfahren sind somit Silikongele in einem definierten Vernetzungsbereich zugänglich, welche den technischen Effekt aufweisen, dass sie zu einem besonders samtig/seidigen Hautgefühl führen und dabei gleichzeitig ein öliges / wachsiges Hautgefühl reduzieren. Somit sind die Silikongele erhältlich nach dem erfindungsgemäßen Verfahren ebenfalls Gegenstand der vorliegenden Erfindung.

Erfindungsgemäß bevorzugte Silikongele weisen bei einer Scherrate von 1 1/s und einer Temperatur von 25 °C eine Viskosität von kleiner 150000 mPas, bevorzugt kleiner 100000 mPas, besonders bevorzug kleiner 50000 mPas insbesondere bevorzugt kleiner 15000 mPas auf. Die Viskosität kann hierbei durch scherratenkontrollierte Rheologie-Experimente bei einer Temperatur von 25°C gemessen werden. Hierfür kann beispielsweise ein Rheometer des Typs MCR 302 der Firma Anton-Paar ausgerüstet mit einer Platte-Platte Messgeometrie eingesetzt werden.

Erfindungsgemäß bevorzugte Silikongele sind zudem dadurch gekennzeichnet, dass sie im frequenzabhängigen Rheologietest bei 25°C im Frequenzbereich von 1-100 Hz ein Speichermodul G‘ aufweisen, welches stets größer ist als das Verlustmodul G". G‘ und G" sind hierbei dem Fachmann geläufige rheologische Kenngrößen und quantifizieren den elastischen bzw. viskosen Anteil von viskoelastischen Flüssigkeiten und werden beispielsweise in Rheokinetics: Rheological Transformations in Synthesis and Reactions of Oligomers and Polymers, A. Ya. Malkin, 1996, beschrieben.

Dies bedeutet, dass die erfindungsgemäßen Silikongele ein ausgeprägte Fließgrenze aufweisen, was sich beispielsweise Vorteilhaft auf deren verdickenden Eigenschaften sowie deren Fähigkeit dispergierte Bestandteile kosmetische Formulierungen zu stabilisieren auswirkt. Für Frequenzabhängige Rheologie-Tests können hierbei beispielsweise ein Rheometer des Typs MCR 302 der Firma Anton-Paar ausgerüstet mit einer Platte-Platte Messgeometrie eingesetzt werden. Besonders bevorzugte Silikongele zeichnen sich dadurch aus, dass sie bei einer Scherrate von 1 1/s und einer Temperatur von 25 °C eine Viskosität von kleiner 10000 mPas aufweisen gleichzeitig im frequenzabhängigen Rheologietest im Frequenzbereich von 1-100 Hz ein Speichermodul G’ aufweisen, welches stets größer als das Verlustmodul G". Entsprechende Silikongele sind hierbei durch eine gute Fließfähigkeit gekennzeichnet, was sich vorteilhaft auf deren Handhabbarkeit und Verarbeitbarkeit auswirkt, weisen aber dennoch eine ausgeprägt Fließgrenze und somit gute verdickende und stabilisierende Eigenschaften auf.

Da die erfindungsgemäßen Silikongele hervorragende Konditioniereigenschaften auf der Haut entfalten, sind kosmetische Formulierungen enthaltend mindestens ein erfindungsgemäßes Silikongel ein weiterer Gegenstand der vorliegenden Erfindung.

Bevorzugte erfindungsgemäße Formulierungen sind dadurch gekennzeichnet, dass sie 1 Gew.-% bis 40 Gew.-%, bevorzugt 2 Gew.-% bis 30 Gew.-%, besonders bevorzugt 3 Gew.-% bis 24 Gew.-%, Silikongel, bezogen auf die Gesamtformulierung, enthalten.

Erfindungsgemäße kosmetische Pflege- und Reinigungsformulierungen, können zum Beispiel mindestens eine zusätzliche Komponente enthalten, ausgewählt aus der Gruppe der Tenside,

Emollients,
Emulgatoren,
Co-Emulgatoren
VerdickerNiskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
Farbpigmente,
kosmetische Wirkstoffe,
Pflegeadditive,
Überfettungsmittel,
Lösungsmittel,
UV-Filter,
Elektrolyte,
multifunktionelle Additive,
feuchtigkeitsspendende Stoffe.

Substanzen, die als beispielhafte Vertreter der einzelnen Gruppen eingesetzt werden können, sind dem Fachmann bekannt und können beispielsweise der EP2273966A1 entnommen werden. Diese Patentanmeldung wird hiermit als Referenz eingeführt und gilt somit als Teil der Offenbarung. Bezüglich weiterer fakultativer Komponenten sowie der eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung. Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Da die erfindungsgemäßen Silikongele sich vorteilhaft zur Herstellung von Reinigungs- und Pflegeformulierungen für Haushalt und Industrie verwenden lassen, sind Reinigungs- und Pflegeformulierungen für Haushalt und Industrie enthaltend mindestens ein erfindungsgemäßes Silikongel ein weiterer Gegenstand der vorliegenden Erfindung.

Bevorzugte erfindungsgemäße Reinigungs- und Pflegeformulierungen für Haushalt und Industrie sind textilweichmachende Formulierungen und textilpflegende Wasch- oder Reinigungsmittel, Geschirrspülmittel, Haushaltsreiniger, Desinfektionsmittel, Desinfektionsreiniger, Schaumreiniger, Fußbodenreiniger, Teppichreiniger, Polsterreiniger, Fußbodenpflegeprodukte, Marmorreiniger, Parkettreiniger, Stein- und Keramikbodenreiniger, Wischpflegemittel, Edelstahlreiniger, Glasreiniger, Geschirrspülmittel, Kunststoffreiniger, Sanitärreiniger, Holzreiniger, Lederreiniger, Waschmittel, Wäschepflegemittel Desinfektionswaschmittel, Vollwaschmittel, Feinwaschmittel, Wollwaschmittel, Weichspülmittel und Imprägniermittel, wobei Geschirrspülmittel und Haushaltsreinigungsmitteln, insbesondere Handgeschirrspülmittel besonders bevorzugt sind. Bevorzugte erfindungsgemäße Reinigungs- und Pflegeformulierungen für Haushalt und Industrie sind dadurch gekennzeichnet, dass sie 1 Gew.-% bis 40 Gew.-%, bevorzugt 2 Gew.-% bis 30 Gew.-%, besonders bevorzugt 3 Gew.-% bis 24 Gew.-%, Silikongel, bezogen auf die Gesamtformulierung, enthalten.

Besonders bevorzugte erfindungsgemäße Reinigungs- und Pflegeformulierungen für Haushalt und Industrie enthalten zusätzlich einen oder mehrere Stoffe aus der Gruppe der Tenside, Gerüststoffe, Bleichmittel, Bleichaktivatoren, Enzyme, Parfüme, Parfümträger, Fluoreszenzmittel, Farbstoffe, Schauminhibitoren, Silikonöle, Antiredepositionsmittel, optische Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Konservierungsmittel, Korrosionsinhibitoren, Antistatika, Bittermittel, Bügelhilfsmittel, Phobier- und Imprägniermittel, Quell- und Schiebefestmittel, neutrale Füllsalze sowie UV-Absorber.

Insbesondere können die erfindungsgemäßen Reinigungs- und Pflegeformulierungen für Haushalt und Industrie zwischen 0,001 und 90 Gew.-%, besonders bevorzugt 0,01 bis 45 Gew.-% eines oder mehrerer der hier genannten weiteren Inhaltsstoffe enthalten, wobei sich die Gew.-% auf die gesamte Formulierung beziehen.

Beispiele für einsetzbare Tenside werden in der WO 2007/115872, Seite 17, Zeile 28 bis Seite 21, Zeile 24 beschrieben.

Beispiele für Gerüststoffe, Builder, Bleichmittel, Bleichaktivatoren, Bleichkatalysatoren und Enzyme werden in der WO 2007/115872, Seite 22, Zeile 7 bis Seite 25, Zeile 26 beschrieben. Antiredepositionsmittel, optische Aufheller, Vergrauungsinhibitoren, Farbübertragungsinhibitoren werden beispielhaft in der WO 2007/115872 auf Seite 26, Zeile 15 bis Seite 28, Zeile 2 beschrieben.

Beispiele von Knitterschutzmitteln, antimikrobiellen Wirkstoffen, Germiziden, Fungiziden, Antioxidantien, Konservierungsmitteln, Antistatika, Bügelhilfsmitteln, UV-Absorbern werden in der WO 2007/115872 auf den Seiten 28, Zeile 14 bis Seite 30, Zeile 22 beschrieben. Deren diesbezüglicher expliziter Offenbarungsgehalt wird durch diese Bezugnahme Teil dieser Offenbarung.

Die erfindungsgemäßen Silikongele und/oder die erfindungsgemäßen Formulierungen lassen sich vorteilhaft zur Konditionierung von Haut und Haar verwenden, was somit ebenfalls ein Gegenstand der vorliegenden Erfindung darstellt.

Weitere vorteilhafte Verwendungen der erfindungsgemäßen Silikongele und/oder der erfindungsgemäßen Formulierungen umfassen kosmetische Anwendungen zum Beispiel aus den Bereichen der Farbkosmetik, Deodorantien, Feuchttücher, Hautpflege für Gesicht und Körper, Haarpflege und Duschprodukte. Hier können die erfindungsgemäßen Silikongele eingesetzt werden als Bindemittel und/oder Dispergierhilfsmittel, zur Beeinflussung des sensorischen Profils während des Auftragens der Formulierung oder nach erfolgter Absorption auf der Haut (Samtigkeit/ Seidigkeit-, Weichheit-, Glattheit-, Gleitfähigeit-verbessernd, Öligkeit-reduzierend, langanhaltendes Hautgefühl), zur Erhöhung der Verfügbarkeit von Wirkstoffen (z.B. Vitamine, Duftstoffe, öllösliche UV-Filter) aus einer Formulierung, zur Erhöhung des Sonnenschutzfaktors, als Mattierungshilfsmittel, für feuchtigkeitsspendende Eigenschaften, als Filmbildner, und diese Verwendungen sind ebenfalls Gegenstand der vorliegenden Erfindung.

Generell können die erfindungsgemäßen Silikongele aufgrund ihrer rheologischen Eigenschaften zur Verdickung von Formulierungen, vor allem zur Verdickung der Ölphase in W/O-Emulsionen, verwendet werden. Die dabei resultierenden Formulierungen zeichnen sich durch eine ausgezeichnete Formulierungsstabilität aus, zum Beispiel im Vergleich zu identischen Formulierungen enthaltend kosmetische Wachse, Xanthan Gum, Silica, Zinc Stearate, Propylene Carbonate und/oder Stearalkonium Hectorite.

In den nachfolgend aufgeführten Beispielen wird die vorliegende Erfindung beispielhaft beschrieben, ohne

dass die Erfindung, deren Anwendungsbreite sich aus der gesamten Beschreibung und den Ansprüchen ergibt, auf die in den Beispielen genannten Ausführungsformen beschränkt sein soll.

Beispiele:

*In den Beispielen eingesetzte Materialien*

Tegosoft® M ist ein Isopropyl-Myristat, welches von der Firma Evonik Industries als kosmetisches Emollient vertrieben wird.

Tegosoft® INI ist ein Isononyl-Isononanoat, welches von der Firma Evonik Industries als kosmetisches Emollient vertrieben wird.

Tegosoft® DEC ist ein Diethylhexyl Carbonate, welches von der Firma Evonik Industries als kosmetisches Emollient vertrieben wird.

Tegosoft® AC ist ein Isoamyl-Cocoate, welches von der Firma Evonik Industries als kosmetisches Emollient vertrieben wird.

Tegosoft® PIB 5 Basic ist ein Polyisobuten, welches von der Firma Evonik Industries als kosmetisches Emollient vertrieben wird.

*Erfindungsgemäßes Beispiel 1: Silikongele hergestellt aus einem Silikonmethacrylat mit einem mittleren molaren Verhältnis von Methacrylatgruppen zu Si-Atomen von 0,055*

Für die Herstellung dieser erfindungsgemäßen Silikongele wurde ein Silikon gemäß Formel (II) mit a2 = 2, b = 170, b2 = 8, $R^1 = CH_3$, $R^3 = -C_6H_{12}-O-C(O)-C(CH_3)=CH_2$ sowie a = a1 = b1 = c = c1 = c2 = d = 0 eingesetzt. 12 g dieses Silikons wurden in einem 250 ml Dreihalskolben, ausgerüstet mit KPG-Rührwerk, Rückflusskühler und Thermometer zusammen mit 87,5 g eines kosmetischen Öls vorgelegt und homogenisiert. Zur Initiierung der Polymerisation wurden der Reaktionsmischung je 0,5 g eines organischen Radikalstarters zugesetzt. Anschließend wurde die Reaktionsmischung unter Stickstoffatmosphäre und gelindem Rühren auf 80 °C erwärmt und für zwei Stunden bei dieser Temperatur gehalten. Zur Nachreaktion wurde die Mischung für eine weitere Stunde auf 100 °C erwärmt. Anschließend wurde die Reaktionsmischung auf Raumtemperatur abgekühlt.

Tabelle 1: Reaktionszusammensetzung für die Herstellung der in Beispiel 1 beschriebenen erfindungsgemäßen Silikongele

| Probenname | Öl-Phase | Radikalstarter |
|---|---|---|
| SG 1 | Tegosoft INI | Dibenzoylperoxid |
| SG 2 | Tegosoft INI | tert-Butylperbenzoat |
| SG 3 | Tegosoft INI | Dilauroylperoxid |
| SG 4 | Tegosoft INI | Azo-bis-(isobutyronitril) |
| SG 5 | Tegosoft DEC | Dilauroylperoxid |
| SG 6 | Tegosoft DEC | Dibenzoylperoxid |

In einer alternativen Herstellungsmethode wurden die in Tabelle 1 zusammengestellten Silikongele durch langsames Zutropfen des Radikalstarters zu einer erwärmten Silikonmakromonomer-Öl-Mischung hergestellt. Hierzu wurden 12 g des Silikons gelöst in 60 g des jeweiligen kosmetischen Öls in einem 250 ml Vierhalskolben, ausgerüstet mit KPG-Rührwerk, Rückflusskühler, Tropftrichter und Thermometer vorgelegt und unter gelindem Rühren auf 80 °C erwärmt. Zu dieser Mischung wurde langsam eine Lösung von 0,5 g Radikalstarter in 27,5 g des jeweiligen kosmetischen Öls über einen Zeitraum von 30 min. zugetropft. Nach der Zugabe wurde die Reaktionsmischung für weitere 90 min. bei dieser Temperatur gehalten und danach zur Nachreaktion für weitere 60 min. auf 100 °C aufgeheizt. Anschließend wurde die Reaktionsmischung auf Raumtemperatur abgekühlt.

In einem weiteren alternativen Herstellverfahren wurden die in Tabelle 1 beschriebenen Silikongele durch langsames Zutropfen einer Radikalstarter - Silikonmakromonomer-Mischung in ein erwärmtes kosmetisches Öl hergestellt. Hierzu wurden 87,5 g des kosmetischen Öls in einem 250 ml Vierhalskolben, ausgerüstet mit KPG-Rührwerk, Rückflusskühler, Tropftrichter und Thermometer vorgelegt und unter gelindem Rühren auf 80 °C erwärmt. Zu diesem Öl wurden langsam 0,5 g eines organischen Radikalstarters gelöst in 12 g des Silikons über einen Zeitraum von 30 min. zugetropft. Nach der Zugabe wurde die Reaktionsmischung für weitere 90 min. bei dieser Temperatur gehalten und danach zur Nachreaktion für weitere 60 min. auf 100 °C aufgeheizt. Anschließend wurde die Reaktionsmischung auf Raumtemperatur abgekühlt.

Für diese Silikongele konnte im Sensoriktest ein samtig-seidiges Hautgefühl festgestellt werden, welches auch nach längerer Zeit nach Applikation fühlbar war.

*Erfindungsgemäßes Beispiel 2: Silikongele hergestellt aus einem Silikonmethacrylat mit einem mittleren molaren Verhältnis von Methacrylatgruppen zu Si-Atomen von 0,047*

Für die Herstellung dieser Erfindungsgemäßen Silikongele wurde ein Silikon gemäß Formel (II) mit a2 = 2, b = 83, $R^1 = CH_3$, $R^3 = (CH_2)_3\text{-}O\text{-}CH_2\text{-}C(CH_2\text{-}O\text{-}C(O)\text{-}C(CH_3){=}CH_2)_2\text{-}CH_2\text{-}CH_3$ sowie a = a1 = b1 = b2 = c = c1 = c2 = d = 0 eingesetzt. Dieses wurde gemäß der in Beispiel 1 beschrieben Reaktionsführungen in ausgewählten kosmetischen Ölen polymerisiert. Je nach Konzentration des Silikons wurden der Reaktionsmischung 0,1 bis 0,8 g Radikalstarter zur Initiierung der Polymerisation zugesetzt. Die genauen Zusammensetzungen der jeweiligen Reaktionsansätze sind in Tabelle 2 zusammengestellt. Ähnlich wie die in Beispiel 1 beschriebenen Gele zeichnen sich die so hergestellten Silikongele im Sensoriktest durch ein samtig-seidiges Hautgefühl aus.

Tabelle 2: Reaktionszusammensetzung für die Herstellung der in Beispiel 2 beschriebenen erfindungsgemäßen Silikongele

| Probenname | Einwaage Silikon [g] | Öl-Phase | Einwaage [g] | Radikalstarter |
|---|---|---|---|---|
| SG 7 | 5,00 | Tegosoft PIB 5 Basic | 95,00 | Dilauroylperoxid |
| SG 8 | 10,00 | Tegosoft PIB 5 Basic | 90,00 | Dilauroylperoxid |
| SG 9 | 15,00 | Tegosoft PIB 5 Basic | 85,00 | Dilauroylperoxid |
| SG 10 | 5,00 | Tegosoft DEC | 95,00 | Dilauroylperoxid |
| SG 11 | 10,00 | Tegosoft DEC | 90,00 | Dilauroylperoxid |
| SG 12 | 15,00 | Tegosoft DEC | 85,00 | Dilauroylperoxid |
| SG 13 | 5,00 | Tegosoft PIB 5 Basic | 95,00 | Dibenzoylperoxid |
| SG 14 | 10,00 | Tegosoft PIB 5 Basic | 90,00 | Dibenzoylperoxid |
| SG 15 | 15,00 | Tegosoft PIB 5 Basic | 85,00 | Dibenzoylperoxid |
| SG 16 | 5,00 | Tegosoft DEC | 95,00 | Dibenzoylperoxid |
| SG 17 | 10,00 | Tegosoft DEC | 90,00 | Dibenzoylperoxid |
| SG 18 | 15,00 | Tegosoft DEC | 85,00 | Dibenzoylperoxid |

*Erfindungsgemäßes Beispiel 3: Silikongele hergestellt aus einem hexadecyl-modifizierten Silikonmethacrylat mit einem mittleren molaren Verhältnis von Methacrylatgruppen zu Si-Atomen von 0,045*

Für die Herstellung dieser Erfindungsgemäßen Silikongele wurde ein Silikon gemäß Formel (II) a2 = 2, b = 83, b1 = 4, $R^1 = CH_3$, $R^3 = (CH_2)_3\text{-}O\text{-}CH_2\text{-}C(CH_2\text{-}O\text{-}C(O)\text{-}C(CH_3){=}CH_2)_2\text{-}CH_2\text{-}CH_3$, $R^2$ = Hexadecyl sowie a = a1 = b2 = c = c1 = c2 = d = 0 eingesetzt. Dieses wurde gemäß der in Beispiel 1 beschrieben Reaktionsführungen in ausgewählten kosmetischen Ölen polymerisiert. Je nach Konzentration des Silikons wurden der Reaktionsmischung 0,1 bis 0,8 g Radikalstarter zur Initiierung der Polymerisation zugesetzt. Die genauen Zusammensetzungen der jeweiligen Reaktionsansätze sind in Tabelle 3 zusammengestellt. Die so hergestellten Silikongele zeichneten sich durch eine sehr hohe Verträglichkeit des Silikonpolymers zum Trägermedium aus. Zudem konnte im Sensoriktest ein besonders samtig-seidiges Hautgefühl dieser Silikongele festgestellt werden.

Tabelle 3: Reaktionszusammensetzung für die Herstellung der in Beispiel 3 beschriebenen erfindungsgemäßen Silikongele

| Probenname | Einwaage Silikon [g] | Öl-Phase | Einwaage [g] | Radikalstarter |
|---|---|---|---|---|
| SG 19 | 5,00 | Tegosoft PIB 5 Basic | 95,00 | Dilauroylperoxid |

(fortgesetzt)

| Probenname | Einwaage Silikon [g] | Öl-Phase | Einwaage [g] | Radikalstarter |
|---|---|---|---|---|
| SG 20 | 7,00 | Tegosoft PIB 5 Basic | 93,00 | Dilauroylperoxid |
| SG 21 | 9,00 | Tegosoft PIB 5 Basic | 91,00 | Dilauroylperoxid |
| SG 22 | 13,00 | Tegosoft PIB 5 Basic | 87,00 | Dilauroylperoxid |
| SG 23 | 5,00 | Tegosoft DEC | 95,00 | Dilauroylperoxid |
| SG 24 | 7,00 | Tegosoft DEC | 93,00 | Dilauroylperoxid |
| SG 25 | 9,00 | Tegosoft DEC | 91,00 | Dilauroylperoxid |
| SG 26 | 13,00 | Tegosoft DEC | 87,00 | Dilauroylperoxid |
| SG 27 | 5,00 | Tegosoft M | 95,00 | Dibenzoylperoxid |
| SG 28 | 7,00 | Tegosoft M | 93,00 | Dibenzoylperoxid |
| SG 29 | 9,00 | Tegosoft M | 91,00 | Dibenzoylperoxid |
| SG 30 | 13,00 | Tegosoft M | 87,00 | Dibenzoylperoxid |
| SG 31 | 5,00 | Tegosoft AC | 95,00 | Dilauroylperoxid |
| SG 32 | 7,00 | Tegosoft AC | 93,00 | Dilauroylperoxid |
| SG 33 | 9,00 | Tegosoft AC | 91,00 | Dilauroylperoxid |
| SG 34 | 13,00 | Tegosoft AC | 87,00 | Dilauroylperoxid |

Zur Charakterisierung des Fließverhaltens diese Silikongele wurden exemplarisch Rheologiemessungen an der Proben SG 20, SG 21 und SG 22 durchgeführt. Viskositätsmessungen bei einer Scherrate von 1 1/s ergaben hierbei folgende Viskositäten von 8000 mPas für die Probe SG 20, 25000 mPas für die Probe SG 21 und 120000 mPas für die Probe SG 22. Frequenzabhängige Rheologiemessungen zeigten zudem, dass für alle diese Proben im kompletten Frequenzbereich von 1 - 100 Hz das Speichermodul G' stets deutlich über dem Verlustmodul G" liegt. Alle Rheologie-Messungen wurden mit einem Rheometer des Typs MCR 302 der Firma Anton-Paar ausgerüstet mit einer Platte-Platte Messgeometrie bei einer Temperatur von 25 °C durchgeführt.

*Nicht erfindungsgemäßes Beispiel 4: Silikongele hergestellt aus einem Silikonmethacrylat mit einem mittleren molaren Verhältnis von Methacrylatgruppen zu Si-Atomen von 0,25*

Zur Herstellung dieser Silikongele wurde ein Silikon gemäß Formel (II) a = 2, b = 13, b2 = 5, $R^1 = CH_3$, $R^3 = (CH_2)_3\text{-O-}CH_2\text{-CH(OH)-}CH_2\text{-O-C(O)-C(}CH_3\text{)=}CH_2$ sowie a1 = a2 = b1 = c = c1 = c2 = d = 0 eingesetzt. Dieses wurde gemäß der in Beispiel 1 beschrieben Reaktionsführungen in ausgewählten kosmetischen Ölen polymerisiert. Je nach Konzentration des Silikons wurden der Reaktionsmischung 0,1 bis 0,8 g Radikalstarter zur Initiierung der Polymerisation zugesetzt. Die genauen Zusammensetzungen der jeweiligen Reaktionsansätze sind in Tabelle 4 zusammengestellt. Bedingt durch die hohe Vernetzungsdichte des Silikonpolymers konnte eine schlechte Verträglichkeit des Silikonpolymers mit dem Trägermedium beobachtet werden, was zunächst zu einer Verklumpung des Gels und schließlich zur Phasenseparation der Probe nach mehreren Tagen Lagerzeit führte. Im Gegensatz zu den in den erfindungsgemäßen Beispielen 1-3 beschriebenen Silikongelen konnte im Sensoriktest ein stumpfes, wenig samtig-seidiges Hautgefühl festgestellt werden.

Tabelle 4: Reaktionszusammensetzung für die Herstellung der in Beispiel 4 beschriebenen nichterfindungsgemäßen Silikongele

| Probenname | Einwaage Silikon [g] | Öl-Phase | Einwaage [g] | Radikalstarter |
|---|---|---|---|---|
| SG 35 | 7,00 | Tegosoft M | 93,00 | Dilauroylperoxid |
| SG 36 | 13,00 | Tegosoft M | 87,00 | Dilauroylperoxid |
| SG 37 | 7,00 | Tegosoft M | 93,00 | tert-Butylperbenzoat |
| SG 38 | 13,00 | Tegosoft M | 87,00 | tert-Butylperbenzoat |
| SG 39 | 7,00 | Tegosoft PIB 5 Basic | 93,00 | Dibenzoylperoxid |

(fortgesetzt)

| Probenname | Einwaage Silikon [g] | Öl-Phase | Einwaage [g] | Radikalstarter |
|---|---|---|---|---|
| SG 40 | 13,00 | Tegosoft PIB 5 Basic | 87,00 | Dibenzoylperoxid |
| SG 41 | 7,00 | Tegosoft PIB 5 Basic | 93,00 | Dilauroylperoxid |
| SG 42 | 13,00 | Tegosoft PIB 5 Basic | 87,00 | Dilauroylperoxid |

*Nicht erfindungsgemäßes Beispiel 5: Silikongele hergestellt aus einem hexadecly-modifizierten Silikonmethacrylat mit einem mittleren molaren Verhältnis von Methacrylatgruppen zu Si-Atomen von 0,013*

Zur Herstellung dieser Silikongele wurde ein Silikon gemäß Formel (II) mit a2 = 2, b = 150, b1 = 3, $R^1 = CH_3$, $R^3 = -C_6H_{12}-O-C(O)-C(CH_3)=CH_2$, $R^2$ = Hexadecyl sowie a = a1 = b2 = c = c1 = c2 = d = 0 eingesetzt. Dieses wurden gemäß der in Beispiel 1 beschrieben Reaktionsführungen in ausgewählten kosmetischen Ölen polymerisiert. Je nach Konzentration des Silikons wurden der Reaktionsmischung 0,1 bis 1,5 g Radikalstarter zur Initiierung der Polymerisation zugesetzt. Die genauen Zusammensetzungen der jeweiligen Reaktionsansätze sind in Tabelle 5 zusammengestellt. Bedingt durch die geringe Vernetzungsdichte des Silikonpolymers konnte ein merklicher Einfluss der Silikongele auf das Hautgefühl erst bei erhöhten Konzentrationen des Silikonpolymers festgestellt werden. Bei diesen erhöhten Konzentrationen war jedoch auffällig, dass die Silikongele vor allem zu einem sehr öligen / fettigem sensorischem Empfinden auf der Haut führten.

Tabelle 5: Reaktionszusammensetzung für die Herstellung der in Beispiel 5 beschriebenen nicht erfindungsgemäßen Silikongele

| Probenname | Einwaage Silikon [g] | Öl-Phase | Einwaage [g] | Radikalstarter |
|---|---|---|---|---|
| SG 43 | 8,00 | Tegosoft AC | 92,00 | Dilauroylperoxid |
| SG 44 | 10,00 | Tegosoft AC | 90,00 | Dilauroylperoxid |
| SG 45 | 13,00 | Tegosoft AC | 87,00 | Dilauroylperoxid |
| SG 46 | 20,00 | Tegosoft AC | 80,00 | Dilauroylperoxid |
| SG 47 | 25,00 | Tegosoft AC | 75,00 | Dilauroylperoxid |
| SG 48 | 30,00 | Tegosoft AC | 70,00 | Dilauroylperoxid |

*Erfindungsgemäßes Beispiel 6: Silikongele hergestellt aus einem verzweigten, hexadecylmodifizierten Silikonmethacrylat mit einem mittleren molaren Verhältnis von Methacrylatgruppen zu Si-Atomen von 0,043*

Für die Herstellung dieser Erfindungsgemäßen Silikongele wurde ein Silikon gemäß Formel (II) a = 2, a2 = 2, b = 83, b1 = 4, c = 2, $R^1 = R^5 = CH_3$, $R^3 = (CH_2)_3-O-CH_2-C(CH_2-O-C(O)-C(CH_3)=CH_2)_2-CH_2-CH_3$, $R^2$ = Hexadecyl sowie a1 = b2 = c1 = c2 = d = 0 eingesetzt. Dieses wurde gemäß der in Beispiel 1 beschrieben Reaktionsführungen in ausgewählten kosmetischen Ölen polymerisiert. Je nach Konzentration des Silikons wurden der Reaktionsmischung 0,1 bis 0,8 g Radikalstarter zur Initiierung der Polymerisation zugesetzt. Die genauen Zusammensetzungen der jeweiligen Reaktionsansätze sind in Tabelle 6 zusammengestellt. Ähnlich wie in Beispiel 3 zeichnen sich die so hergestellten Silikongele durch eine sehr hohe Verträglichkeit des Silikonpolymers zum Trägermedium aus. Zudem konnte im Sensoriktest ein besonders samtig-seidiges Hautgefühl dieser Silikongele festgestellt werden.

Tabelle 6: Reaktionszusammensetzung für die Herstellung der in Beispiel 4 beschriebenen nichterfindungsgemäßen Silikongele

| Probenname | Einwaage Silikon [g] | Öl-Phase | Einwaage [g] | Radikalstarter |
|---|---|---|---|---|
| SG 49 | 7,00 | Tegosoft M | 93,00 | Dilauroylperoxid |
| SG 50 | 13,00 | Tegosoft M | 87,00 | Dilauroylperoxid |

(fortgesetzt)

| Probenname | Einwaage Silikon [g] | Öl-Phase | Einwaage [g] | Radikalstarter |
|---|---|---|---|---|
| SG 51 | 7,00 | Tegosoft PIB 5 Basic | 93,00 | Dilauroylperoxid |
| SG 52 | 13,00 | Tegosoft PIB 5 Basic | 87,00 | Dilauroylperoxid |

*Vergleichsbeispiel 7: Sensorische Evaluierung von kosmetischen Formulierungen enthaltend erfindungsgemäßes Silikongel aus Beispiel 3 im Vergleich zu nichterfindungsgemäßem Silikongel aus Vergleichsbeispiel 4*

Es wurden die in Tabelle 6 gezeigten Formulierungen hergestellt. Der Einfluss auf das Hautgefühl der Formulierungen wurde durch einen Paneltest untersucht. Sechs Personen applizierten jeweils eine definierte Menge von ca. 25 μL der beiden Formulierungen auf ein definiertes Testfeld auf der Innenseite des Unterarms, ohne die Zusammensetzung der Formulierungen zu kennen. Die Formulierungen wurden mithilfe eines Fingers durch kreisende Bewegungen in dem Testfeld verteilt. Nach erfolgter Absorption wurde pausiert und nach 5 Minuten das Hautgefühl auf dem Testfeld beurteilt.

Die Ergebnisse der Beurteilung des Hautgefühls sind am Ende der Tabelle 7 dargestellt. Es werden die Eigenschaften aufgeführt, die eine Mehrheit der Personen als bevorzugt, nach Auftragung einer der beiden Formulierungen im Vergleich zur Auftragung der anderen Formulierung, beschrieben hat.

Tabelle 7: Formulierungen zu Vergleichsbeispiel 6, Wasser-in-Öl Emulsionen. Klassische W/O Heiss-/Kalt Lotions-Herstellung. Phase A wurde bei 85°C für 1 min bei 20500 rpm mit dem Ultraturrax dispergiert.

| | **AL 2/14** | **9a** | **9b** |
|---|---|---|---|
| A | ISOLAN® GPS (Polyglyceryl-4 Diisostearate/Polyhydroxystearate/Sebacate) | 3,0 | 3,0 |
| | **Silikongel aus erfindungsgemäßem Bsp. 3 (SG 22)** | **7,0** | |
| | **Silikongel aus nichterfindungsgemäßem Bsp. 4 (SG 42)** | | **7,0** |
| | TEGOSOFT® DEC (Diethylhexyl Carbonate) | | |
| | TEGOSOFT® AC (Isoamyl Cocoate) | | |
| | TEGOSOFT® PIB 5 Basic (Polybutene) | | |
| | Cyclopentasiloxane | 10,8 | 10,8 |
| | Hydrogenated Castor Oil | 0,1 | 0,1 |
| | Paracera™ W 80 Wax (Paramelt) (Paraffin, Microcristallina cera) | 0,1 | 0,1 |
| B | Wasser | 74,8 | 74,8 |
| | Glycerin | 2,0 | 2,0 |
| | Magnesium Sulfate Heptahydrate | 1,5 | 1,5 |
| C | Euxyl® PE 9010 (Schülke & Mayr) (Phenoxyethanol, Ethylhexylglycerin) | 0,7 | 0,7 |
| | Hautgefühl nach 5 Minuten | weniger wachsig, gleitfähiger, samtiger/ seidiger | wachsiger, weniger gleitfähig, weniger samtig/ seidig |

Das Hautgefühl nach Auftragung der Formulierung enthaltend Silikongel aus erfindungsgemäßem Beispiel 3 im Vergleich zu Silikongel aus nicht erfindungsgemäßem Beispiel 4 ist weniger wachsig, gleitfähiger. Insgesamt wird ein wachsigeres und weniger gleitfähiges Hautgefühl als stumpf beschrieben. Auch die Samtigkeit/Seidigkeit nach Auftragung der Formulierung enthaltend Silikongel aus erfindungsgemäßem Beispiel 3 im Vergleich zu Silikongel aus nicht erfindungsgemäßem Beispiel 4 ist verbessert.

*Vergleichsbeispiel 8: Sensorische Evaluierung von kosmetischen Formulierungen enthaltend erfindungsgemäßes Silikongel aus Beispiel 3 im Vergleich zu nichterfindungsgemäßem Silikongel aus Vergleichsbeispiel 5*

Es wurden die in Tabelle 8 gezeigten Formulierungen hergestellt. Der Einfluss auf das Hautgefühl der Formulierungen wurde durch einen Paneltest untersucht. Acht Personen applizierten jeweils eine definierte Menge von ca. 25 μL der beiden Formulierungen auf ein definiertes Testfeld auf der Innenseite des Unterarms, ohne die Zusammensetzung der Formulierungen zu kennen. Die Formulierungen wurden mithilfe eines Fingers durch kreisende Bewegungen in dem Testfeld verteilt. Nach erfolgter Absorption wurde die Öligkeit durch optische und taktile Prüfung auf den Testfeldern beurteilt.

Die Ergebnisse der Beurteilung des Parameters Öligkeit sind am Ende der Tabelle 8 dargestellt. Es werden die Eigenschaften aufgeführt, die eine Mehrheit der Personen als bevorzugt, nach Auftragung einer der beiden Formulierungen im Vergleich zur Auftragung der anderen Formulierung, beschrieben hat.

Tabelle 8: Formulierungen zu Vergleichsbeispiel 7, Öl-in-Wasser Emulsionen. Klassische O/W Heiss-/Heiss Creme-Herstellung.

| | **FU 08/12** | **5a** | **5b** |
|---|---|---|---|
| A | TEGO® Care 450 (Polyglyceryl-3 Methylglucose Distearate) | 3,0 | 3,0 |
| | TEGIN® M Pellets (Glyceryl Stearate) | 2,0 | 2,0 |
| | TEGO® Alkanol 18 (Stearyl Alcohol) | 1,0 | 1,0 |
| | TEGOSOFT® OS (Ethylhexyl Stearate) | 3,5 | 3,5 |
| | TEGOSOFT®CT (Caprylic/Capric Triglyceride) | 3,5 | 3,5 |
| | **Silikongel aus erfindungsgemäßem Bsp. 3 (SG 34)** | **12,0** | |
| | **Silikongel aus nichterfindungsgemäßem Bsp. 5 (SG 45)** | | **12,0** |
| B | Wasser | 74.2 | 74.2 |
| C | Methylisothiazolinone, Methylparaben, Ethylparaben (Microcare® MEM, Thor Personal Care) | 0.8 | 0.8 |
| | Hautgefühl nach Absorption | weniger ölig | öliger |

Die Öligkeit auf der Haut ist nach Auftragung der Formulierung enthaltend Silikongel aus erfindungsgemäßem Beispiel 3 im Vergleich zu Silikongel aus nicht erfindungsgemäßem Beispiel 5 reduziert.

*Vergleichsbeispiel 9: Verbesserte Stabilität von kosmetischen Formulierungen unter Verwendung erfindungsgemäßer Silikongele als Verdicker*

Die erfindungsgemäßen Silikongele können als Verdicker eingesetzt werden, vor allem zur Verdickung der Ölphase in W/O-Emulsionen. Die dabei resultierenden Formulierungen zeichnen sich durch eine ausgezeichnete Stabilität aus, vor allem hinsichtlich der Gefrier-/Taustabilität und sind dadurch gegenüber auf andere Weise verdickten Formulierung bevorzugt.

Im direkten Vergleich wurde in einer kosmetischen W/O-Emulsion die verdickenden Eigenschaften der erfindungsgemäßen Silikongele mit denen von kosmetischen Wachsen, Silica, Zinc Stearate und einem Gemisch bestehend aus Dicaprylyl Carbonate/ Stearalkonium Hectorite/ Propylene Carbonate verglichen.

Tabelle 9: Formulierungen zu Vergleichsbeispiel 8. Klassische W/O-Herstellung. *Phase A wurde für 1 min bei 20500 rpm mit dem Ultraturrax dispergiert.

| | **AL 2/14** | **76** | **81** | **86*** | **82** | **87*** | **89*** |
|---|---|---|---|---|---|---|---|
| A | ABIL® EM 90 (Cetyl PEG/PPG-10/1 Dimethicone) | 2 | 2 | 2 | 2 | 2 | 2 |

(fortgesetzt)

| | AL 2/14 | 76 | 81 | 86* | 82 | 87* | 89* |
|---|---|---|---|---|---|---|---|
| | **Silikongel aus erfindungsgemäßem Bsp. 3 (SG 20)** | | **12** | | **8** | | |
| | TEGOSOFT® OP (Ethylhexyl Palmitate) | 5 | 2,8 | 4,4 | 3,6 | 5 | 5,1 |
| | TEGOSOFT® CT (Caprylic/Capric Triglyceride) | 10 | 5,6 | 8,8 | 7,2 | 10 | 10,2 |
| | TEGOSOFT® DEC (Diethylhexyl Carbonate) | 10 | 5,6 | 8,8 | 7,2 | 10 | 10,2 |
| | Paracera™ W 80 Wax (Paramelt) (Paraffin, Microcristallina cera) | 0,5 | | | | | |
| | Hydrogenated Castor Oil | 0,5 | | | | | |
| | AEROSIL® R 972 (Silica) | | | | | 1 | |
| | Zinc Stearate | | | | | | 0,5 |
| | Cosmedia Gel CC (Cognis) (Dicaprylyl Carbonate, Stearalkonium Hectorite, Propylene Carbonate) | | | 4 | | | |
| B | Wasser | 70,7 | 70,7 | 70,7 | 70,7 | 70,7 | 70,7 |
| | Sodium Chloride | 0,5 | 0,5 | 0,5 | 0,5 | 0,5 | 0,5 |
| C | Euxyl® PE 9010 (Schülke & Mayr) (Phenoxyethanol, Ethylhexylglycerin) | 0,8 | 0,8 | 0,8 | 0,8 | 0,8 | 0,8 |
| | Viskosität [Pas] | 15 | 15 | 15 | 7 | 7 | 6 |

Dazu wurden Formulierungen mit vergleichbarer Viskosität hergestellt. Diese wurden auf ihre Gefrier-/Taustabilität hin überprüft indem die Formulierungen bei -15°C über Nacht gelagert wurden, dann auf Raumtemperatur gebracht wurden, und dieser Vorgang insgesamt 3 mal wiederholt wurde. In den Fällen, in denen die W/O-Emulsion mit kosmetischen Wachsen, Silica, Zinc Stearate oder einem Gemisch bestehend aus Dicaprylyl Carbonate/ Stearalkonium Hectorite/ Propylene Carbonate hergestellt wurden zeigte sich nach den 3 Gefrier-/Tauzyklen Wasserabscheidun. Diese trat in den Fällen, in denen die W/O-Emulsionen mit den erfindungsgemäßem Silikongel verdickt wurden, nicht auf.

*Vergleichsbeispiel 10: Verarbeitbarkeit*

Bedingt durch ihre noch relative gute Fließfähigkeit ist ein weiterer Vorteil der erfindungsgemäßen Silikongele ihre gute Handhabbarkeit sowie Verarbeitbarkeit. Dadurch heben sie sich von anderen marktüblichen Silikongelen ab, welche teilweise sehr hohe Viskositäten aufweisen, nicht pumpbar sind und aus dem Gebinde gekratzt und bei der Verarbeitung unter Einfluß hoher Scherkräfte homogenisiert bzw. dispergiert werden müssen.

Tabelle 10: Formulierungen zu Vergleichsbeispiel 9. Klassische W/O-Herstellung. *Phase A wurde für 1 min bei 20500 rpm mit dem Ultraturrax dispergiert.

| | AL 2/14 | 32* | 33* | 35* | 36* | 37* | 38* | 41 | 42 |
|---|---|---|---|---|---|---|---|---|---|
| A | ISOLAT® GPS (Polyglyceryl-4 Diisostearate/Polyhydroxystearate/ Sebacate) | 3,0 | 3,0 | 3,0 | 3,0 | 3,0 | 3,0 | 3,0 | 3,0 |
| | **Silikongel aus erfindungsgemäßem Bsp. 3 (SG 21)** | | | | | | | **3,0** | |
| | **Silikongel aus erfindungsgemäßem Bsp. 3 (SG 25)** | | | | | | | | **3,0** |

(fortgesetzt)

| | AL 2/14 | 32* | 33* | 35* | 36* | 37* | 38* | 41 | 42 |
|---|---|---|---|---|---|---|---|---|---|
| | 9041 Silicone Elastomer Blend (Dow Corning) (Dimethicone, Dimethicone Crosspolymer) | | 5,0 | | | | | | |
| | KSG-16 (Shin-Etsu) (Dimethicone, Dimethicone/Vinyl Dimethicone Crosspolymer) | | | 3,0 | | | | | |
| | 9040 Silicone Elastomer Blend (Dow Corning) (Cyclopentasiloxane, Dimethicone Crosspolymer) | | | | 3,0 | | | | |
| | KSG-15 (Shin-Etsu) (Cyclopentasiloxane, Dimethicone/Vinyl Dimethicone Crosspolymer) | 5,0 | | | | | | | |
| | Velvesil DM (Momentive) (Dimethicone, Cetearyl Dimethicone Crosspolymer) | | | | | 3,0 | | | |
| | Silsoft Silicone Gel (Cyclopentasiloxane, Cetearyl Dimethicone/Vinyl Dimethicone | | | | | | 3,0 | | |
| | Crosspolymer; Momentive) | | | | | | | | |
| | Cyclopentasiloxane | 6,4 | 6,4 | 7,4 | 7,4 | 7,4 | 7,4 | 7,4 | 7,4 |
| | TEGOSOFT® DEC (Diethylhexyl Carbonate) | 6,4 | 6,4 | 7,4 | 7,4 | 7,4 | 7,4 | 7,4 | 7,4 |
| | Hydrogenated Castor Oil | 0,1 | 0,1 | 0,1 | 0,1 | 0,1 | 0,1 | 0,1 | 0,1 |
| | Paracera™ W 80 Wax (Paramelt) (Paraffin, Microcristallina cera) | 0,1 | 0,1 | 0,1 | 0,1 | 0,1 | 0,1 | 0,1 | 0,1 |
| B | Wasser | 74,8 | 74,8 | 74,8 | 74,8 | 74,8 | 74,8 | 74,8 | 74,8 |
| | Glycerin | 2,0 | 2,0 | 2,0 | 2,0 | 2,0 | 2,0 | 2,0 | 2,0 |
| | Magnesium Sulfate Heptahydrate | 1,5 | 1,5 | 1,5 | 1,5 | 1,5 | 1,5 | 1,5 | 1,5 |
| C | Euxyl® PE 9010 (Schülke & Mayr) (Phenoxyethanol, Ethylhexylglycerin) | 0,7 | 0,7 | 0,7 | 0,7 | 0,7 | 0,7 | 0,7 | 0,7 |

Die in diesem Beispiel eingesetzten marktüblichen Silikongele müssen alle mithilfe eines Spatels aus dem Gebinde gekratzt und bei der W/O-Herstellung unter Einfluß hoher Scherkräfte homogenisiert bzw. dispergiert werden. In dem Fall, in dem das erfindungsgemäße Silikongel eingesetzt wurde, konnte dieses einfach durch Pumpen oder Schütten aus dem Gebinde entfernt werden.

*Beispielformulierungen*

Die nachfolgend aufgeführten Formulierungsbeispiele sind in der Mehrzahl Emulsionen vom Typ Öl-in-Wasser (O/W) oder Wasser-in-Öl (W/O). Diese können nach üblichen, dem Fachmann bekannten, Methoden unter Verwendung typischer Rühraggregate hergestellt werden. Vorzugsweise werden W/O-Emulsionen durch langsames Einrühren der Wasserphase in die Ölphase mit nachfolgender Homogenisierung hergestellt. Bei den beschriebenen O/W Emulsionen werden vorzugsweise Öl- und Wasserphase ohne Rühren zusammengeführt und anschließend homogenisiert. Dies kann in einem Kalt-/Kalt-Prozess geschehen oder in einem Heiss-/Heiss-Prozess, bei dem die Homogenisierung bei ca. 70 °C stattfindet. Die erfindungsgemäßen Silikongele können dabei prinzipiell in jeder Stufe des Prozesses eingearbeitet werden. In den meisten Beispielformulierungen erfolgte entweder die Einarbeitung in die Emulsion bei Temperaturen <60 °C oder die Silikongele wurden zusammen mit der Ölphase vorgelegt. Alternativ kann auch in manchen Fällen ein Heiss-/Kalt-Prozess eingesetzt werden, bei dem die Ölphase wie üblich erhitzt wird, die Wasserphase jedoch nicht oder nur sehr milde erhitzt wird.

**W/O Lotion**

| | | % |
|---|---|---|
| A | ISOLAN® GPS (Polyglyceryl-4 Diisostearate / Polyhydroxystearate / Sebacate) | 3.00 |
| | **Silikongel aus erfindungsgemäßem Bsp. 3 (SG 32)** | **8.00** |
| | Cyclopentasiloxane | 4.90 |
| | TEGOSOFT® DEC (Diethylhexyl Carbonate) | 4.90 |
| | Hydrogenated Castor Oil | 0.10 |
| | Paracera™ W 80 Wax (Paramelt) (Paraffin, Microcristallina cera) | 0.10 |
| B | Water | ad 100 |
| | Glycerin | 2.00 |
| | Magnesium Sulfate Heptahydrate | 1.50 |
| C | Euxyl® PE 9010 (Schülke & Mayr) (Phenoxyethanol, Ethylhexylglycerin) | 0.70 |
| D | Perfume | q.s. |

**W/O Lotion**

| | | % |
|---|---|---|
| A | ABIL® EM 120 (Bis-(Glyceryl/Lauryl) Glyceryl Lauryl Dimethicone, Caprylic/Capric Triglyceride) | 3.00 |
| | **Silikongel aus erfindungsgemäßem Bsp. 3 (SG 28)** | **12.00** |
| | Cyclopentasiloxane | 2.90 |
| | TEGOSOFT® P (Isopropyl Palmitate) | 2.90 |
| | Hydrogenated Castor Oil | 0.10 |
| | Paracera™ W 80 Wax (Paramelt) (Paraffin, Microcristallina cera) | 0.10 |
| B | Water | ad 100 |
| | Glycerin | 2.00 |
| | Sodium Chloride | 0.80 |
| C | Euxyl® PE 9010 (Schülke & Mayr) (Phenoxyethanol, Ethylhexylglycerin) | 0.70 |

**W/O Lotion**

| | | % | % | % | % | % | % |
|---|---|---|---|---|---|---|---|
| A | ABIL® EM 90 (Cetyl PEG/PPG-10/1 Dimethicone) | 3.00 | 3.00 | 3.00 | 3.00 | 3.00 | 3.00 |
| | **Silikongel aus erfindungsgemäßem Bsp. 3 (SG 24)** | **4.00** | **8.00** | **12.00** | **16.00** | **20.00** | **24.00** |
| | Cyclopentasiloxane | 26.00 | 22.00 | 18.00 | 14.00 | 10.00 | 6.00 |
| B | Water | ad 100 | ad 100 | ad 100 | ad 100 | ad 100 | ad 100 |
| | Sodium Chloride | 0.50 | 0.50 | 0.50 | 0.50 | 0.50 | 0.50 |
| C | Euxyl® PE 9010 (Schülke & Mayr) (Phenoxyethanol, Ethylhexylglycerin) | 0.70 | 0.70 | 0.70 | 0.70 | 0.70 | 0.70 |

**W/O Lotion**

| | | % | % | % | % | % | % |
|---|---|---|---|---|---|---|---|
| A | ABIL® EM 90 (Cetyl PEG/PPG-10/1 Dimethicone) | 2.00 | 2.00 | 2.00 | 2.00 | 2.00 | 2.00 |
| | **Silikongel aus erfindungsgemäßem Bsp. 3 (SG 20)** | **24.00** | **20.00** | **16.00** | **12.00** | **8.00** | **4.00** |
| | TEGOSOFT® OP (Ethylhexyl Palmitate) | 0.45 | 1.20 | 2.00 | 2.80 | 3.60 | 4.40 |
| | TEGOSOFT® CT (Caprylic/Capric Triglyceride) | 0.90 | 2.40 | 4.00 | 5.60 | 7.20 | 8.80 |
| | TEGOSOFT® DEC (Diethylhexyl Carbonate) | 0.90 | 2.40 | 4.00 | 5.60 | 7.20 | 8.80 |
| B | Water | ad 100 | ad 100 | ad 100 | ad 100 | ad 100 | ad 100 |
| | Sodium Chloride | 0.50 | 0.50 | 0.50 | 0.50 | 0.50 | 0.50 |
| C | Euxyl® PE 9010 (Schülke & Mayr) (Phenoxyethanol, Ethylhexylglycerin) | 0.80 | 0.80 | 0.80 | 0.80 | 0.80 | 0.80 |

**W/O Lotion**

| | | % |
|---|---|---|
| A | ABIL® EM 180 (Cetyl PEG/PPG-10/1 Dimethicone) | 1.50 |
| | **Silikongel aus erfindungsgemäßem Bsp. 3 (SG 23)** | **16.00** |
| | Cyclopentasiloxane | 14.00 |
| B | Water | ad 100 |
| | Sodium Chloride | 0.50 |
| C | Euxyl® PE 9010 (Schülke & Mayr) (Phenoxyethanol, Ethylhexylglycerin) | 0.70 |

**W/O Lotion**

| | | % |
|---|---|---|
| A | ABIL® EM 180 (Cetyl PEG/PPG-10/1 Dimethicone) | 1.00 |
| | **Silikongel aus erfindungsgemäßem Bsp. 3 (SG 29)** | **8.00** |
| | TEGOSOFT® OP (Ethylhexyl Palmitate) | 3.60 |
| | TEGOSOFT® CT (Caprylic/Capric Triglyceride) | 7.20 |
| | TEGOSOFT® DEC (Diethylhexyl Carbonate) | 7.20 |
| B | Water | ad 100 |
| | Sodium Chloride | 0.50 |
| C | Euxyl® PE 9010 (Schülke & Mayr) (Phenoxyethanol, Ethylhexylglycerin) | 0.80 |

**W/O Lotion with Urea**

| | | % |
|---|---|---|
| A | ABIL® EM 180 (Cetyl PEG/PPG-10/1 Dimethicone) | 1.50 |
| | TEGOSOFT® G 20 (Octyldodecanol) | 2.90 |

(fortgesetzt)

| | | % |
|---|---|---|
| | TEGOSOFT® CT (Caprylic/Capric Triglyceride) | 2.20 |
| | TEGOSOFT® P (Isopropyl Palmitate) | 2.20 |
| | Mineral Oil (30mPas) | 3.20 |
| | Paracera™ W 80 Wax (Paramelt) (Paraffin, Microcristallina cera) | 0.50 |
| | Hydrogenated Castor Oil | 0.50 |
| | **Silikongel aus erfindungsgemäßem Bsp. 3 (SG 25)** | **12.00** |
| B | Sodium Chloride | 0.70 |
| | Glycerin | 3.00 |
| | Urea | 5.00 |
| | Triacetin (Glyceryl Triacetate) | 0.01 |
| | Water | ad 100 |
| C | Phenonip XB (Phenoxyethanol, Methylparaben, Propylparaben, Ethylparaben) | 0.70 |

**Anti-Aging Day Care Cream**

| | | % |
|---|---|---|
| A | ABIL® EM 120 (Bis-(Glyceryl/Lauryl) Glyceryl Lauryl Dimethicone, Caprylic/Capric Triglyceride) | 3.00 |
| | TEGOSOFT® AC (Isoamyl Cocoate) | 3.50 |
| | TEGOSOFT® DEC (Diethylhexyl Carbonate) | 3.00 |
| | HyaCare® Filler CL (Water; Ethylhexyl Stearate; Sodium Hyaluronate Crosspolymer; Polyglyceryl-4 Diisostearate/Polyhydroxystearate/Sebacate; Sodium Isostearate) | 2.50 |
| | TEGOLON® ECO 10-10 (Nylon-10/10) | 2.00 |
| | Tocopherol | 0.50 |
| | **Silikongel aus erfindungsgemäßem Bsp. 3 (SG 33)** | **8.00** |
| B | Water | ad 100 |
| | Sodium Chloride | 0.80 |
| | Glycerin | 4.00 |
| | Butylene Glycol | 4.00 |
| | TEGO® Pep 4-Even (Tetrapeptide-30; Glycerin) | 2.50 |
| | Sodium Ascorbyl Phospate | 1.50 |
| | Urea | 2.50 |
| | Sodium Bisulfite (40% in water) | 0.30 |
| C | Euxyl® PE 9010 (Schülke & Mayr) (Phenoxyethanol, Ethylhexylglycerin) | 0.70 |

**Dual-Action Wrinkle Serum**

| | | % |
|---|---|---|
| A | ABIL® EM 90 (Cetyl PEG/PPG-10/1 Dimethicone) | 1.50 |
| | ABIL® EM 97 S (Bis-PEG / PPG-14 / 14 Dimethicone; Dimethicone) | 1.00 |

(fortgesetzt)

| | | % |
|---|---|---|
| | Cyclopentasiloxane | 6.00 |
| | TEGOSOFT® DEC (Diethylhexyl Carbonate) | 5.00 |
| | HyaCare® Filler CL (Water; Ethylhexyl Stearate; Sodium Hyaluronate Crosspolymer; Polyglyceryl-4 Diisostearate/Polyhydroxystearate/Sebacate; Sodium Isostearate) | 2.50 |
| | Tocopherol | 0.50 |
| | Zinc Stearate | 0.50 |
| | **Silikongel aus erfindungsgemäßem Bsp. 3 (SG 22)** | **4.00** |
| B | Water | ad 100 |
| | Glycerin | 4.00 |
| | Butylene Glycol | 4.00 |
| | Sodium Chloride | 0.80 |
| | TEGO® Pep 4-17 (Tetrapeptide-21; Glycerin; Butylene Glycol; Water) | 0.50 |
| C | Euxyl® PE 9010 (Schülke & Mayr) (Phenoxyethanol, Ethylhexylglycerin) | 0.40 |

**Light O/W Anti Aging Lotion (Cold Processing)**

| | | % |
|---|---|---|
| A | TEGO® Care LTP (Sorbitan Laurate; Polyglyceryl-4 Laurate; Dilauryl Citrate) | 1.50 |
| | TEGOSOFT® CI (Cetearyl Isononanoate) | 2.50 |
| | TEGOSOFT® DEC (Diethylhexyl Carbonate) | 1.60 |
| | TEGOSOFT® OP (Ethylhexyl Palmitate) | 0.50 |
| | TEGO® Carbomer 140 (Carbomer) | 0.15 |
| | TEGO® Carbomer 141 (Carbomer) | 0.15 |
| | Xanthan Gum | 0.10 |
| | **Silikongel aus erfindungsgemäßem Bsp. 3 (SG 27)** | **10.00** |
| B | TEGO® Cosmo C 100 (Creatine) | 0.50 |
| | Glycerin | 3.00 |
| | Water | ad 100 |
| C | Sodium Hydroxide (10% in water) | 0.90 |
| | Phenonip XB (Phenoxyethanol, Methylparaben, Propylparaben, Ethylparaben) | 0.70 |

O/W Body Lotion

| | | % |
|---|---|---|
| A | AXOL® C 62 Pellets (Glyceryl Stearate Citrate) | 1.50 |
| | TEGO® Alkanol 1618 (Cetearyl Alcohol) | 1.00 |
| | TEGOSOFT® CT (Caprylic/Capric Triglyceride) | 3.90 |
| | TEGOSOFT® MM (Myristyl Myristate) | 1.60 |
| | Tocopheryl Acetate | 1.00 |

(fortgesetzt)

| | | |
|---|---|---|
| | | % |
| | **Silikongel aus erfindungsgemäßem Bsp. 3 (SG 19)** | **8.00** |
| B | Glycerin | 5.00 |
| | GluCare® S (Sodium Carboxymethyl Beta-Glucan) | 0.20 |
| | Water | ad 100 |
| C | TEGO® Carbomer 141 (Carbomer) | 0.20 |
| | TEGOSOFT® OS (Ethylhexyl Stearate) | 0.80 |
| D | Sodium Hydroxide (10% in water) | 0.60 |
| E | Alcohol | 3.00 |
| F | Microcare MEM (Dipropylene Glycol; Methylparaben; Ethylparaben; Water; Methylisothiazolinone) | 0.70 |

**Light and refreshing O/W Lotion**

| | | |
|---|---|---|
| | | % |
| A | **Silikongel aus erfindungsgemäßem Bsp. 3 (SG 31)** | **12.00** |
| | ABIL® 350 (Dimethicone) | 0.80 |
| | TEGOSOFT® DEC (Diethylhexyl Carbonate) | 2.00 |
| | ABIL® Care XL 80 (Bis-PEG/PPG-20/5 PEG/PPG-20/5 Dimethicone; Methoxy PEG/PPG-25/4 Dimethicone; Caprylic/Capric Triglyceride) | 2.00 |
| B | Glycerin | 1.00 |
| | Water | ad 100 |
| C | TEGO® Carbomer 341 ER (Carbomer) | 0.25 |
| | TEGOSOFT® DEC (Diethylhexyl Carbonate) | 1.00 |
| D | Sodium Hydroxide (10% in water) | 0.70 |
| E | Euxyl® PE 9010 (Schülke & Mayr) (Phenoxyethanol, Ethylhexylglycerin) | 1.00 |

**O/W Lotion with Light Skin Feel**

| | | |
|---|---|---|
| | | % |
| A | TEGO® Care PSC 3 (Polyglyceryl-3 Dicitrate/Stearate) | 2.50 |
| | TEGOSOFT® AC (Isoamyl Cocoate) | 3.80 |
| | TEGOSOFT® CT (Caprylic/Capric Triglyceride) | 3.00 |
| | Prunus Amygdalus Dulcis (Sweet Almond) Oil | 2.70 |
| | **Silikongel aus erfindungsgemäßem Bsp. 3 (SG 24)** | **8.00** |
| B | Water | ad 100 |
| | Glycerin | 3.00 |
| C | Xanthan Gum | 0.50 |
| D | Sodium Hydroxide (10% in water) | 0.20 |
| E | Rokonsal BSB-N (Benzyl Alcohol; Glycerin; Benzoic Acid; Sorbic Acid) | 0.80 |

**Wellness Body Lotion**

| | | % |
|---|---|---|
| A | TEGO® Care PSC 3 (Polyglyceryl-3 Dicitrate/Stearate) | 3.00 |
| | TEGOSOFT® CT (Caprylic/Capric Triglyceride) | 2.70 |
| | TEGOSOFT® OER (Oleyl Erucate) | 2.00 |
| | Prunus Amygdalus Dulcis (Sweet Almond) Oil | 3.30 |
| | **Silikongel aus erfindungsgemäßem Bsp. 3 (SG 34)** | **4.00** |
| B | Water | ad 100 |
| | Glycerin | 3.00 |
| C | Xanthan Gum | 0.50 |
| D | Sodium Hydroxide (10% in water) | 0.20 |
| E | Rokonsal BSB-N (Benzyl Alcohol; Glycerin; Benzoic Acid; Sorbic Acid) | 0.80 |

**O/W Lotion with peptide**

| | | % |
|---|---|---|
| A | TEGO® Care PSC 3 (Polyglyceryl-3 Dicitrate/Stearate) | 3.00 |
| | TEGOSOFT® OP (Ethylhexyl Palmitate) | 5.30 |
| | TEGOSOFT® M (Isopropyl Myristate) | 2.60 |
| | TEGOSOFT® CI (Cetearyl Isononanoate) | 1.10 |
| | **Silikongel aus erfindungsgemäßem Bsp. 3 (SG 20)** | **8.00** |
| B | Water | ad 100 |
| | Glycerin | 3.00 |
| | TEGO® Pep 4-17 (Tetrapeptide-21; Glycerin; Butylene Glycol; Water) | 2.50 |
| C | TEGO® Carbomer 141 (Carbomer) | 0.20 |
| | TEGOSOFT® OP (Ethylhexyl Palmitate) | 0.80 |
| D | Sodium Hydroxide (10% in water) | 0.60 |
| E | Microcare MEM (Dipropylene Glycol; Methylparaben; Ethylparaben; Water; Methylisothiazolinone) | 0.80 |

**O/W Cream**

| | | % |
|---|---|---|
| A | TEGO® Care 450 (Polyglyceryl-3 Methylglucose Distearate) | 3.00 |
| | TEGIN® M Pellets (Glyceryl Stearate) | 2.00 |
| | TEGO® Alkanol 18 (Stearyl Alcohol) | 1.00 |
| | TEGOSOFT® OS (Ethylhexyl Stearate) | 4.50 |
| | TEGOSOFT® CT (Caprylic/Capric Triglyceride) | 4.50 |
| | **Silikongel aus erfindungsgemäßem Bsp. 3 (SG 28)** | **10.00** |
| B | Water | ad 100 |
| | Glycerin | 3.00 |
| C | Euxyl® PE 9010 (Schülke & Mayr) (Phenoxyethanol, Ethylhexylglycerin) | 1.00 |

**Anti-Aging O/W Cream**

| | | % |
|---|---|---|
| A | TEGO® Care 450 (Polyglyceryl-3 Methylglucose Distearate) | 3.00 |
| | TEGIN® M Pellets (Glyceryl Stearate) | 2.00 |
| | TEGO® Alkanol 18 (Stearyl Alcohol) | 1.00 |
| | TEGOSOFT® OS (Ethylhexyl Stearate) | 3.50 |
| | TEGOSOFT® CT (Caprylic/Capric Triglyceride) | 3.50 |
| | **Silikongel aus erfindungsgemäßem Bsp. 3 (SG 23)** | **12.00** |
| | Ceramide III (Ceramide NP) | 0.10 |
| B | Water | ad 100 |
| | Glycerin | 3.00 |
| | TEGO® PEP 4-17 (Tetrapeptide-21; Glycerin; Butylene Glycol; Water) | 2.50 |
| | HyaCare® 50 (Hydrolyzed Hyaluronic Acid) | 0.10 |
| C | Euxyl® PE 9010 (Schülke & Mayr) (Phenoxyethanol, Ethylhexylglycerin) | 1.00 |

**Time Zone Anti-Aging Cream**

| | | % |
|---|---|---|
| A | TEGO® Care 450 (Polyglyceryl-3 Methylglucose Distearate) | 3.00 |
| | TEGIN® M Pellets (Glyceryl Stearate) | 2.00 |
| | TEGO® Alkanol 18 (Stearyl Alcohol) | 1.00 |
| | TEGOSOFT® CT (Caprylic/Capric Triglyceride) | 3.50 |
| | TEGOSOFT® TN (C12-15 Alkyl Benzoate) | 3.50 |
| | **Silikongel aus erfindungsgemäßem Bsp. 3 (SG 19)** | **12.00** |
| B | TEGO® Pep 4-17 (Tetrapeptide-21; Glycerin; Butylene Glycol; Water) | 4.00 |
| | HyaCare® (Sodium Hyaluronate) | 0.10 |
| | HyaCare® 50 (Hydrolyzed Hyaluronic Acid) | 0.10 |
| | Glycerin | 3.00 |
| | Water | ad 100 |
| D | HyaCare® Filler CL (Water; Ethylhexyl Stearate; Sodium Hyaluronate Crosspolymer; Polyglyceryl-4 Diisostearate/ Polyhydroxystearate/Sebacate; Sodium Isostearate) | 5.00 |
| E | Microcare MEM (Dipropylene Glycol; Methylparaben; Ethylparaben; Water; Methylisothiazolinone) | 0.80 |

**O/W Cream**

| | | % |
|---|---|---|
| A | TEGO® Care 450 (Polyglyceryl-3 Methylglucose Distearate) | 3.00 |
| | TEGIN® M Pellets (Glyceryl Stearate) | 1.50 |
| | TEGO® Alkanol 18 (Stearyl Alcohol) | 1.50 |
| | TEGOSOFT® OS (Ethylhexyl Stearate) | 4.50 |

(fortgesetzt)

| | | % |
|---|---|---|
| | TEGOSOFT® CT (Caprylic/Capric Triglyceride) | 3.70 |
| | **Silikongel aus erfindungsgemäßem Bsp. 3 (SG 32)** | **10.00** |
| B | Water | ad 100 |
| | Glycerin | 3.00 |
| C | TEGO® Carbomer 134 (Carbomer) | 0.20 |
| | TEGOSOFT® CT (Caprylic/Capric Triglyceride) | 0.80 |
| D | Euxyl® PE 9010 (Schülke & Mayr) (Phenoxyethanol, Ethylhexylglycerin) | 1.00 |

**O/W Cream**

| | | % |
|---|---|---|
| A | TEGO® Care 165 (Glyceryl Stearate; PEG-100 Stearate) | 6.00 |
| | TEGIN® M Pellets (Glyceryl Stearate) | 2.00 |
| | TEGO® Alkanol 18 (Stearyl Alcohol) | 1.00 |
| | TEGOSOFT® OS (Ethylhexyl Stearate) | 4.50 |
| | TEGOSOFT® CT (Caprylic/Capric Triglyceride) | 4.50 |
| | **Silikongel aus erfindungsgemäßem Bsp. 3 (SG 27)** | **10.00** |
| B | Water | ad 100 |
| | Glycerin | 3.00 |
| C | Microcare MEM (Dipropylene Glycol; Methylparaben; Ethylparaben; Water; Methylisothiazolinone) | 0.80 |

**Anti-Aging O/W Cream**

| | | % |
|---|---|---|
| A | TEGO® Care 165 (Glyceryl Stearate; PEG-100 Stearate) | 6.00 |
| | TEGIN® M Pellets (Glyceryl Stearate) | 2.00 |
| | TEGO® Alkanol 18 (Stearyl Alcohol) | 1.00 |
| | TEGOSOFT® OS (Ethylhexyl Stearate) | 5.50 |
| | TEGOSOFT® CT (Caprylic/Capric Triglyceride) | 5.50 |
| | **Silikongel aus erfindungsgemäßem Bsp. 3 (SG 21)** | **8.00** |
| | Ceramide III (Ceramide NP) | 0.10 |
| B | Water | ad 100 |
| | Glycerin | 3.00 |
| | TEGO® PEP 4-17 (Tetrapeptide-21; Glycerin; Butylene Glycol; Water) | 2.50 |
| | HyaCare® 50 (Hydrolyzed Hyaluronic Acid) | 0.10 |
| C | Microcare MEM (Dipropylene Glycol; Methylparaben; Ethylparaben; Water; Methylisothiazolinone) | 0.80 |

**O/W Urea Softcream**

| | | % |
|---|---|---|
| A | TEGO® Care PSC 3 (Polyglyceryl-3 Dicitrate/Stearate) | 3.00 |
| | TEGIN® M Pellets (Glyceryl Stearate) | 1.50 |
| | TEGO® Alksnol 1618 (Cetearyl Alcohol) | 1.00 |
| | TEGOSOFT® CT (Caprylic/Capric Triglyceride) | 3.90 |
| | TEGOSOFT® OS (Ethylhexyl Stearate) | 2.00 |
| | TEGOSOFT® DC (Decyl Cocoate) | 1.60 |
| | **Silikongel aus erfindungsgemäßem Bsp. 3 (SG 26)** | **4.00** |
| B | Water | ad 100 |
| | Glycerin | 3.00 |
| C | Water | 10.00 |
| | Urea | 10.00 |
| | Triacetin (Glyceryl Triacetate) | 0.01 |
| D | Microcare MEM (Dipropylene Glycol; Methylparaben; Ethylparaben; Water; Methylisothiazolinone) | 0.80 |

**O/W Eye Cream with low SPF (UVA)**

| | | % |
|---|---|---|
| A | TEGO® Care PSC 3 (Polyglyceryl-3 Dicitrate/Stearate) | 3.00 |
| | TEGIN® M Pellets (Glyceryl Stearate) | 2.50 |
| | TEGO® Alkanol 18 (Stearyl Alcohol) | 1.50 |
| | TEGOSOFT® CT (Caprylic/Capric Triglyceride) | 0.30 |
| | TEGOSOFT® XC (Phenoxyethyl Caprylate) | 4.00 |
| | TEGOSOFT® TIS (Triisostearin) | 1.00 |
| | TEGOSOFT® CR (Cetyl Ricinoleate) | 0.50 |
| | HyaCare® Filler CL (Water; Ethylhexyl Stearate; Sodium Hyaluronate Crosspolymer; Polyglyceryl-4 Diisostearate/ Polyhydroxystearate/Sebacate; Sodium Isostearate) | 3.00 |
| | Persea Gratissima (Avocado) Oil | 2.00 |
| | Octocylene | 4.00 |
| | Ethylhexyl Methoxycinnamate | 2.00 |
| | Diethylamino Hydroxybenzoyl Hexyl Benzoate | 1.70 |
| | Tocopheryl Acetate | 0.50 |
| | **Silikongel aus erfindungsgemäßem Bsp. 3 (SG 30)** | **4.00** |
| B | Water | ad 100 |
| | Glycerin | 3.00 |
| C | Microcare MEM (Dipropylene Glycol; Methylparaben; Ethylparaben; Water; Methylisothiazolinone) | 0.80 |

**Si/W Emulsion**

| | | % |
|---|---|---|
| A | **Silikongel aus erfindungsgemäßem Bsp. 3 (SG 20)** | **10.00** |
| | ABIL® 350 (Dimethicone) | 0.80 |
| | Dimethicone (Wacker Belsil DM 5) | 2.00 |
| | ABIL® Care XL 80 (Bis-PEG/PPG-20/5 PEG/PPG-20/5 Dimethicone; Methoxy PEG/PPG-25/4 Dimethicone; Caprylic/Capric Triglyceride) | 2.00 |
| B | Glycerin | 1.00 |
| | Water | ad 100 |
| C | TEGO® Carbomer 341 ER (Carbomer) | 0.25 |
| | Dimethicone (Wacker Belsil DM 5) | 1.00 |
| D | Sodium Hydroxide (10% in water) | 0.70 |
| E | Euxyl® PE 9010 (Schülke & Mayr) (Phenoxyethanol, Ethylhexylglycerin) | 1.00 |

**Men's Care Gel**

| | | % |
|---|---|---|
| A | TEGOSOFT® OER (Oleyl Erucate) | 1.00 |
| | TEGOSOFT® TIS (Triisostearin) | 1.00 |
| | **Silikongel aus erfindungsgemäßem Bsp. 3 (SG 22)** | **4.00** |
| B | Water | ad 100 |
| | Glycerin | 5.00 |
| | TEGO® Carbomer 341 ER (Carbomer) | 0.50 |
| | HyaCare® 50 (Hydrolyzed Hyaluronic Acid) | 0.10 |
| C | Sodium Hydroxide (10% in water) | 1.50 |
| D | Microcare MEM (Dipropylene Glycol; Methylparaben; Ethylparaben; Water; Methylisothiazolinone) | 0.80 |

**Deodorant Cream for sensitive skin**

| | | % |
|---|---|---|
| A | TEGO® Care PBS 6 (Polyglyceryl-6 Stearate, Polyglyceryl-6 Behenate) | 3.00 |
| | TEGIN® M Pellets (Glyceryl Stearate) | 1.00 |
| | TEGO® Alkanol 18 (Stearyl Alcohol) | 2.00 |
| | TEGOSOFT® E (PPG-15 Stearyl Ether) | 11.50 |
| | Persea Gratissima (Avocado) Oil | 2.00 |
| | TEGODEO® PY 88 G (Zinc Ricinoleate) | 1.00 |
| | TEGO® Cosmo P 813 (Polyglyceryl-3 Caprylate) | 0.50 |
| | Triethyl Citrate | 1.00 |
| | **Silikongel aus erfindungsgemäßem Bsp. 3 (SG 33)** | **4.00** |
| B | Natrosol 250 HHR (Hydroxyethyl Cellulose) | 1.00 |
| | Water | ad 100 |
| C | Microcare MEM (Dipropylene Glycol; Methylparaben; Ethylparaben; Water; Methylisothiazolinone) | 0.80 |

**HyaCare® Lip Filler**

| | | % |
|---|---|---|
| A | TEGOSOFT® G 20 (Octyldodecanol) | 12.40 |
| | TEGOSOFT® CT (Caprylic/Capric Triglyceride) | 8.80 |
| | TEGOSOFT® OER (Oleyl Erucate) | 2.80 |
| | TEGOSOFT® MM (Myristyl Myristate) | 5.00 |
| | Ricinus Communis (Castor) Seed Oil | 32.20 |
| | CREMERLIN® pura (Olus (Vegetable) Oil) | 10.00 |
| | BW Ester BW 67 (Stearyl Beeswax; Behenyl Beeswax) | 6.50 |
| | Kahlwax 1899 (Ozokerite) | 6.00 |
| | Kahlwax 8104 (Cera Alba) | 5.00 |
| | Kahlwax 2039L (Euphorbia Cerifera (Candellila) Wax) | 3.00 |
| | **Silikongel aus erfindungsgemäßem Bsp. 3 (SG 25)** | **8.00** |
| B | Tocopherol | 0.10 |
| | Perfume Vanillea | 0.20 |

**W/O Foundation**

| | | % |
|---|---|---|
| A | ABIL® EM 120 (Bis-(Glyceryl/Lauryl) Glyceryl Lauryl Dimethicone, Caprylic/Capric Triglyceride) | 4.00 |
| | TEGOSOFT® XC (Phenoxyethyl Caprylate) | 3.50 |
| | Cyclopentasiloxane | 3.50 |
| | Isododecane | 3.50 |
| | Sicovit Braun 70 E 172 (CI 77491; CI 77492; CI 77499 (Iron Oxides)) | 2.50 |
| | Hombitan AC 360 (CI 77891; Titanium Dioxide; Alumina; Triethyoxycaprylylsilane) | 4.00 |
| | Talc | 2.00 |
| | **Silikongel aus erfindungsgemäßem Bsp. 3 (SG 26)** | **4.00** |
| B | Cosmedia Gel CC (Dicaprylyl Carbonate; Stearalkonium Hectorite; Propylene Carbonate) | 4.00 |
| C | Water | ad 100 |
| | Glycerin | 5.00 |
| | Sodium Chloride | 2.00 |
| D | Boroneige 1201 (Boron Nitride) | 5.00 |
| | Phenonip XB (Phenoxyethanol, Methylparaben, Propylparaben, Ethylparaben) | 0.70 |

**Mattifvina Compact Cream Make-up**

| | | % |
|---|---|---|
| A | TEGOSOFT® AC (Isoamyl Cocoate) | 23.70 |
| | TEGOSOFT® CR (Cetyl Ricinoleate) | 5.00 |
| | TEGOSOFT® OP (Ethylhexyl Palmitate) | 8.00 |
| | ABIL® Wax 9840 (Cetyl Dimethicone) | 1.00 |

(fortgesetzt)

| | | % |
|---|---|---|
| | TEGOSOFT® XC (Phenoxyethyl Caprylate) | 13.00 |
| | Mikrowachs 1899 (Ozokerite) | 2.00 |
| | Kahlwachs 6607 (Helianthus Annuus (Sunflower Seed) Wax) | 4.00 |
| | **Silikongel aus erfindungsgemäßem Bsp. 3 (SG 31)** | **8.00** |
| B | $TiO_2$ Kronos 1171 (Titanium Dioxide) | 12.00 |
| | Sicovit Gelb 10E (Yellow Iron Oxide) | 1.00 |
| | Sicovit Rot 30E (Red Iron Oxide) | 0.30 |
| | Sicovit Braun 70E (Brown Iron Oxide) | 0.60 |
| | Sicovit Schwarz 80E (Black Iron Oxide) | 0.20 |
| | TEGOLON® ECO 10-10 (Nylon-10/10) | 20.00 |
| C | Dermosoft GMCY (Glyceryl Caprylate) | 1.00 |
| | Perfume Sheabutter & Arganöl | 0.20 |

**Color Control Fluid SPF 25**

| | | % |
|---|---|---|
| A | ABIL® EM 120 (Bis-(Glyceryl/Lauryl) Glyceryl Lauryl Dimethicone, Caprylic/Capric Triglyceride) | 4.00 |
| | TEGOSOFT® DEC (Diethylhexyl Carbonate) | 6.20 |
| | Cyclopentasiloxane | 6.00 |
| | Uvinul A Plus B (Ethylhexyl Methoxycinnamate; Diethylamino Hydroxybenzoyl Hexyl Benzoate) | 10.00 |
| | TEGOLON® 12-20 (Nylon-12) | 2.00 |
| | Micro Talc IT Extra-AW (Talc) | 2.00 |
| | Hombitan AC 360 (CI 77891; Titanium Dioxide; Alumina; Triethyoxycaprylylsilane) | 5.00 |
| | Unipure Yellow LC 182 | 0.50 |
| | Unipure Red LC 381 | 0.20 |
| | Unipure Black LC 989 | 0.10 |
| | Timiron Splendid Gold (Titanium Dioxide; Mica; Silica) | 0.25 |
| | **Silikongel aus erfindungsgemäßem Bsp. 3 (SG 29)** | **4.00** |
| B | Cosmedia Gel CC (Dicaprylyl Carbonate; Stearalkonium Hectorite; Propylene Carbonate) | 3.00 |
| C | Water | ad 100 |
| | Glycerin | 3.00 |
| | Sodium Chloride | 1.50 |
| | TEGO® Cosmo C 250 (1-Methylhydantoin-2-Imide) | 1.00 |
| | TEGO® Pep 4-even (Tetrapeptide-30; Glycerin) | 2.00 |
| | TEGO® Stemlastin (Algae Extract) | 1.00 |
| D | Euxyl® K 900 (Schülke & Mayr GmbH) (Benzyl Alcohol, Ethylhexylglycerin, Tocopherol) | 1.00 |

**Skin Beautifier BB Cream SPF 15**

| | | % |
|---|---|---|
| A | TEGO® Care 450 (Polyglyceryl-3 Methylglucose Distearate) | 3.00 |
| | TEGIN® M Pellets (Glyceryl Stearate) | 2.50 |
| | TEGO® Alkanol 18 (Stearyl Alcohol) | 1.50 |
| | TEGOSOFT® DEC (Diethylhexyl Carbonate) | 2.90 |
| | HyaCare® Filler CL (Water; Ethylhexyl Stearate; Sodium Hyaluronate Crosspolymer; Polyglyceryl-4 Diisostearate/ Polyhydroxystearate/Sebacate; Sodium Isostearate) | 2.00 |
| | Phytosphingosine | 0.10 |
| | Ethylhexyl Methoxycinnamate | 5.00 |
| | Diethylamino Hydroxybenzoyl Hexyl Benzoate | 3.00 |
| | **Silikongel aus erfindungsgemäßem Bsp. 3 (SG 34)** | **4.00** |
| B | Hombitan AC 360 (CI 77891; Titanium Dioxide; Alumina; Triethyoxycaprylylsilane) | 3.00 |
| | Micro Talc IT Extra-AW (Talc) | 2.00 |
| | Unipure Yellow LC 182 | 0.36 |
| | Unipure Red LC 381 | 0.12 |
| | Unipure Black LC 989 | 0.08 |
| | TEGOSOFT® AC (Isoamyl Cocoate) | 4.44 |
| | TEGOSOFT® XC (Phenoxyethyl Caprylate) | 4.00 |
| | TEGOLON® ECO 10-10 (Nylon-10/10) | 3.00 |
| C | Water | ad 100 |
| | Glycerin | 3.00 |
| | HyaCare® 50 (Hydrolyzed Hyaluronic Acid) | 0.10 |
| | TEGO® Pep 4-17 (Tetrapeptide-21; Glycerin; Butylene Glycol; Water) | 2.00 |
| D | Alcohol | 3.00 |
| E | Microcare MEM (Dipropylene Glycol; Methylparaben; Ethylparaben; Water; Methylisothiazolinone) | 0.80 |

**Face Powder**

| | | % |
|---|---|---|
| A | Talc | 76.00 |
| | Magnesium Stearate | 2.50 |
| | Ronaflair M Spheres (Mica; Silica) | 10.00 |
| | Sicovit Braun 70E (Brown Iron Oxide) | 3.00 |
| | TEGOLON® ECO 10-10 (Nylon-10/10) | 5.00 |
| B | **Silikongel aus erfindungsgemäßem Bsp. 3 (SG 22)** | **3.00** |
| | Dermosoft LP (Caprylyl Glycol; Glyceryl Caprylate; Glycerin; Phenyl Propanol; Water) | 0.50 |

**Eye Shadow blue**

| | | % |
|---|---|---|
| A | Talc | 43.50 |
| | Magnesium Stearate | 5.00 |
| | Sicovit Schwarz 80E 172 (CI 77499; Black Iron Oxide) | 1.00 |
| | Sicomet Blau P 77007 (CI 77007; Ultramarines) | 14.00 |
| | TEGOLON® ECO 10-10 (Nylon-10/10) | 5.00 |
| B | Colorona Light Blue (Mica; Titanium Dioxide; Ferric Ferrocyanide) | 25.00 |
| C | ABIL® 350 (Dimethicone) | 3.00 |
| | Dermosoft LP (Caprylyl Glycol; Glyceryl Caprylate; Glycerin; Phenyl Propanol; Water) | 0.50 |
| | **Silikongel aus erfindungsgemäßem Bsp. 3 (SG 30)** | **3.00** |

**W/O Sun Lotion with medium SPF**

| | | % |
|---|---|---|
| A | ABIL® EM 90 (Cetyl PEG/PPG-10/1 Dimethicone) | 2.50 |
| | Paracera™ W 80 Wax (Paramelt) (Paraffin, Microcristallina cera) | 0.20 |
| | Hydrogenated Castor Oil | 0.20 |
| | **Silikongel aus erfindungsgemäßem Bsp. 3 (SG 21)** | **10.00** |
| | TEGOSOFT® DEC (Diethylhexyl Carbonate) | 3.80 |
| | TEGOSOFT® CT (Caprylic/Capric Triglyceride) | 4.90 |
| | TEGOSOFT® XC (Phenoxyethyl Caprylate) | 4.90 |
| | Octocrylene | 4.00 |
| | Butyl Methoxydibenzoylmethane | 2.50 |
| | Ethylhexyl Methoxycinnamate | 3.00 |
| | Bis-Ethylhexyloxyphenol Methoxyphenyl Triazine | 2.00 |
| B | Water | ad 100 |
| | Glycerin | 3.00 |
| | Sodium Chloride | 0.50 |
| | Phenylbenzimidazol Sulfonic Acid (20% in water) | 5.00 |
| C | Euxyl® PE 9010 (Schülke & Mayr) (Phenoxyethanol, Ethylhexylglycerin) | 0.70 |

**Low viscous W/O Sun Care Lotion SPF 30**

| | | % |
|---|---|---|
| A | Diethylamino Hydroxybenzoyl Hexyl Benzoate | 9.00 |
| | Ethylhexyl Methoxycinnamate | 8.00 |
| | Octocrylene | 4.00 |
| B | ISOLAN® GPS (Polyglyceryl-4 Diisostearate / Polyhydroxystearate / Sebacate) | 2.00 |
| | TEGOLON® ECO 10-10 (Nylon-10/10) | 2.00 |
| | Sepigel 305 (Polyacrylamide, C13-14 Isoparaffin, Laureth-7) | 1.25 |

(fortgesetzt)

| | | % |
|---|---|---|
| | EUXYL® K 300 (Schülke & Mayr) (Phenoxyethanol; Methylparaben; Ethylparaben; Butylparaben; Propylparaben; Isobutylparaben) | 0.50 |
| | Aerosil R 812 S (Silica Silyate) | 0.50 |
| | **Silikongel aus erfindungsgemäßem Bsp. 3 (SG 34)** | **4.00** |
| C | Water | ad 100 |
| | Glycerin | 5.00 |
| | VARISOFT® 300 (Cetrimonium Chloride) | 1.00 |
| | Alcohol | 0.30 |
| D | Perfume | q.s. |

**O/W Sun Protection Lotion with high SPF**

| | | % |
|---|---|---|
| A | AXOL® C 62 Pellets (Glyceryl Stearate Citrate) | 2.50 |
| | TEGIN® M Pellets (Glyceryl Stearate) | 1.00 |
| | TEGO® Alkanol 1618 (Cetearyl Alcohol) | 0.50 |
| | TEGOSOFT® XC (Phenoxyethyl Caprylate) | 6.00 |
| | Diisopropyl Adipate | 2.00 |
| | Diethylamino Hydroxybenzoyl Hexyl Benzoate | 4.00 |
| | Octocrylene | 8.00 |
| | Butyl Methoxydibenzoylmethane | 1.50 |
| | Ethylhexyl Salicylate | 5.00 |
| | Ethylhexyl Triazone | 2.00 |
| | Ethylhexyl Methoxycinnamate | 2.00 |
| | REWOPAL® PIB 1000 (Polyisobutene) | 2.00 |
| | **Silikongel aus erfindungsgemäßem Bsp. 3 (SG 26)** | **4.00** |
| B | Water | ad 100 |
| | Glycerin | 3.00 |
| C | TEGOSOFT® XC (Phenoxyethyl Caprylate) | 0.80 |
| | TEGO® Carbomer 141 (Carbomer) | 0.20 |
| D | Sodium Hydroxide (10% in water) | 0.60 |
| E | TEGOLON® ECO 10-10 (Nylon-10/10) | 5.00 |
| F | Microcare MEM (Dipropylene Glycol; Methylparaben; Ethylparaben; Water; Methylisothiazolinone) | 0.80 |

**O/W Sun Protection Lotion Cold Processing**

| | | % |
|---|---|---|
| A | TEGO® Care LTP (Sorbitan Laurate; Polyglyceryl-4 Laurate; Dilauryl Citrate) | 2.00 |
| | TEGOSOFT® DC (Decyl Cocoate) | 2.00 |

(fortgesetzt)

| | | % |
|---|---|---|
| | TEGOSOFT® TN (C12-15 Alkyl Benzoate) | 1.50 |
| | TEGOSOFT® OP (Ethylhexyl Palmitate) | 0.60 |
| | Ethylhexyl Methoxycinnamate | 5.00 |
| | Butyl Methoxydibenzoylmethane | 2.00 |
| | Octocrylene | 3.00 |
| | Persea Gratissima (Avocado) Oil | 1.00 |
| | TEGO® Carbomer 141 (Carbomer) | 0.15 |
| | TEGO® Carbomer 140 (Carbomer) | 0.15 |
| | Xanthan Gum | 0.10 |
| | **Silikongel aus erfindungsgemäßem Bsp. 3 (SG 33)** | **4.00** |
| B | Glycerin | 3.00 |
| | Water | ad 100 |
| C | Sodium Hydroxide (10% in water) | 0.90 |
| D | Phenonip XB (Phenoxyethanol, Methylparaben, Propylparaben, Ethylparaben) | 1.00 |

**O/W After Sun Lotion**

| | | % |
|---|---|---|
| A | TEGO® Care PS (Methyl Glucose Sesquistearate) | 2.00 |
| | TEGOSOFT® OS (Ethylhexyl Stearate) | 2.50 |
| | Mineral Oil (30mPas) | 2.50 |
| | Tocopheryl Acetate | 0.50 |
| | **Silikongel aus erfindungsgemäßem Bsp. 3 (SG 21)** | **8.00** |
| B | Glycerin | 3.00 |
| | Water | ad 100 |
| | Panthenol | 0.50 |
| C | Mineral Oil (30mPas) | 0.80 |
| | TEGO® Carbomer 141 (Carbomer) | 0.20 |
| D | Sodium Hydroxide (10% in water) | 0.40 |
| E | Euxyl® PE 9010 (Schülke & Mayr) (Phenoxyethanol, Ethylhexylglycerin) | 1.00 |

**Moisturizina After Sun Lotion**

| | | % |
|---|---|---|
| A | TEGO® Care PBS 6 (Polyglyceryl-6 Stearate, Polyglyceryl-6 Behenate) | 3.00 |
| | TEGIN® M Pellets (Glyceryl Stearate) | 0.35 |
| | TEGO® Alkanol 1618 (Cetearyl Alcohol) | 0.65 |
| | Butyrospermum parkii (Shea) Butter | 3.00 |
| | TEGOSOFT® CT (Caprylic/Capric Triglyceride) | 3.50 |

(fortgesetzt)

| | | % |
|---|---|---|
| | TEGOSOFT® P (Isopropyl Palmitate) | 3.00 |
| | **Silikongel aus erfindungsgemäßem Bsp. 3 (SG 30)** | **4.00** |
| B | Water | ad 100 |
| | Glycerin | 5.00 |
| | Triacetin (Glyceryl Triacetate) | 0.20 |
| C | Xanthan Gum | 0.50 |
| D | Urea (50% in water) | 40.00 |
| E | Euxyl® PE 9010 (Schülke & Mayr) (Phenoxyethanol, Ethylhexylglycerin) | 0.70 |

**Suspension Antiperspirant Roll-on**

| | | % |
|---|---|---|
| A | Cyclopentasiloxane | 57.00 |
| | **Silikongel aus erfindungsgemäßem Bsp. 3 (SG 20)** | **10.00** |
| | Quaternium-18 Hectorite | 11.50 |
| | AEROSIL® R 972 (Silica) | 0.50 |
| | Aluminum Chlorohydrate | 20.00 |
| B | Perfume | 1.00 |

**Suspension Antiperspirant Roll-on**

| | | % |
|---|---|---|
| A | Cyclopentasiloxane, Disteardimonium Hectorite, Propylene Carbonate (Bentone Gel® VS-5 PC V) | 3.00 |
| | Cyclopentasiloxane | 60.00 |
| | **Silikongel aus erfindungsgemäßem Bsp. 3 (SG 24)** | **10.00** |
| | ABIL® 100 (Dimethicone) | 4.00 |
| | Ethanol | 2.00 |
| B | Aluminum Zirconium Tetrachlorohydrex GLY (Reach AZP-908 O) | 20.00 |
| C | Perfume | 1.00 |

## Patentansprüche

**1.** Verfahren zur Herstellung von Silikongelen umfassend die Verfahrensschritte

A) Bereitstellen einer Mischung umfassend
ein Silikon enthaltend
mindestens eine (Meth-)Acrylat-Gruppe der Formel (I)

$$-O-C(O)-CR=CH_2 \qquad \text{Formel (I)}$$

mit R = -H oder $-CH_3$,
mit der Maßgabe, dass das mittlere molare Verhältnis von (Meth-)AcrylatGruppen zu Si Atomen im Silikon in einem Bereich von 0,014 - 0,15 liegt, und

mindestens ein kosmetisch geeignetes Trägermedium,
B) Initiieren einer radikalischen Polymerisation durch Zugabe mindestens eines organischen Radikalstarters, bevorzugt eines organischen Peroxids.

**2.** Verfahren gemäß Anspruch 1, **dadurch gekennzeichnet, dass** das eingesetzte Silikon mindestens eine Alkylseitenketten mit 2 bis 30, bevorzugt 8 bis 24, besonders bevorzugt 12 bis 18, C-Atomen aufweist.

**3.** Verfahren gemäß Anspruch 1 oder 2, **dadurch gekennzeichnet, dass** die Konzentration des Silikons in der Silikon-Öl-Mischung von 1 bis 40 Gew.-%, bevorzugt von 3 bis 30 Gew.-%, besonders bevorzugt von 5 bis 25 Gew.-%, bezogen auf das Gesamtgewicht der Silikon-Öl-Mischung beträgt.

**4.** Verfahren gemäß mindestens einem der vorgenannten Ansprüche, **dadurch gekennzeichnet, dass** das mittlere molare Verhältnis von (Meth-)Acrylat-Gruppen zu Si Atomen im Silikon im Bereich von 0,016 bis 0,12, besondere bevorzugt von 0,018 bis 0,10, insbesondere bevorzugt von 0,020 bis 0,075, liegt.

**5.** Verfahren gemäß mindestens einem der vorgenannten Ansprüche, **dadurch gekennzeichnet, dass** das Silikon verzweigte oder unverzweigte Alkylreste aufweist, welche sich von langkettigen Fettsäuren ableiten, insbesondere Cetyl.

**6.** Verfahren gemäß mindestens einem der vorgenannten Ansprüche, **dadurch gekennzeichnet, dass** das Silikon einem der allgemeinen Formel (II) entspricht

$$M_a\, M'_{a1}\, M''_{a2}\, D_b\, D'_{b1}\, D''_{b2}\, T_c\, T'_{c1}\, T''_{c2}\, Q_d \qquad \text{Formel (II),}$$

wobei

$M = (R^1_3\ Si\ O_{1/2})$
$M' = (R^2R^1_2\ Si\ O_{1/2})$
$M'' = (R^3R^1_2\ Si\ O_{1/2})$
$D = (R^1_2\ Si\ O_{2/2})$
$D' = (R^2R^1\ Si\ O_{2/2})$
$D'' = (R^3R^1\ Si\ O_{2/2})$
$T = (R^5\ Si\ O_{3/2})$
$T' = (R^2\ Si\ O_{3/2})$
$T'' = (R^3\ Si\ O_{3/2})$
$Q = (Si\ O_{4/2})$
a = 0 bis 32; bevorzugt 0 bis 22, insbesondere >= 2;
a1 = 0 bis 32, bevorzugt 0 bis 22, insbesondere 0;
a2 = 0 bis 32; bevorzugt 0 bis 22, insbesondere >= 2;

mit der Maßgabe, dass

$$a + a1 + a2 >= 2, \text{ bevorzugt} = 2;$$

b = 1 bis 600, bevorzugt 10 bis 500, insbesondere 20 bis 400;
b1 = 0 bis 80, bevorzugt 0 bis 50, insbesondere 1 bis 20;
b2 = 0 bis 50, bevorzugt 0 bis 20, insbesondere 0 bis 10;
c = 0 bis 30, bevorzugt 0 bis 20, insbesondere 0;
c1 = 0 bis 10, bevorzugt 0 bis 5, insbesondere 0;
c2 = 0 bis 10, bevorzugt 0 bis 5, insbesondere 0;
d = 0 bis 15, bevorzugt 0 bis 12, insbesondere 0;

mit der Maßgabe, dass

$$a2 + b2 + c2 >= 1$$

und

$R^1$ = unabhängig voneinander gleiche oder verschiedene lineare oder verzweigte, gegebenenfalls aromatische Kohlenwasserstoffreste mit 1 bis 30 Kohlenstoffatomen, bevorzugt mit 1 bis 6 Kohlenstoffatomen, besonders bevorzugt Methyl oder Phenyl, insbesondere Methyl;
$R^2$ = unabhängig voneinander gleiche oder verschiedene lineare oder verzweigte, gegebenenfalls aromatische Kohlenwasserstoffreste mit 2 bis 30, bevorzugt 7 bis 22, Kohlenstoffatomen, bevorzugt lineare gesättigte Kohlenwasserstoffreste mit 6 bis 30, bevorzugt 7 bis 22, Kohlenstoffatomen, besonders bevorzugt lineare gesättigte Kohlenwasserstoffreste mit 8 bis 22, insbesondere 12 bis 18 Kohlenstoffatomen, insbesondere Dodecyl-, Tetradecyl-, Hexadecyl- und Octadecylreste;
$R^3$ = unabhängig voneinander gleiche oder verschiedene organische Reste, die eine (Meth-)Acrylat-Gruppe der Formel (I)

$$-O-C(O)-CR=CH_2 \qquad \text{Formel (I)}$$

mit R = -H oder $-CH_3$,
aufweisen;
$R^5$ = unabhängig voneinander gleiche oder verschiedene Reste $R^1$, $R^2$ oder $R^3$, bevorzugt $R^1$, insbesondere Methyl, Phenyl, Dodecyl oder Hexadecyl,

sind.

7. Verfahren gemäß mindestens einem der vorgenannten Ansprüche, **dadurch gekennzeichnet, dass** ein Silikon der allgemeinen Formel (II) eingesetzt wird mit

$a + a1 + a2 = 2$ und $c + c1 + c2 + d = 0$ oder
$a2 = 2$ und $c + c1 + c2 + d = 0$.

8. Verfahren gemäß mindestens einem der vorgenannten Ansprüche, **dadurch gekennzeichnet, dass** ein Silikon der allgemeinen Formel (II) eingesetzt wird mit

a2 = 2, b = 20 bis 400, b2 = 1 bis 20, $R^1 = CH_3$, $R^3 = -C_6H_{,2}-O-C(O)-C(CH_3)=CH_2$, sowie a = a1 = b1 = c = c1 = c2 = d = 0;
a2 = 2, b = 20 bis 400, $R^1 = CH_3$, $R^3 = (CH_2)_3-O-CH_2-C(CH_2-O-C(O)-C(CH_3)=CH_2)_2-CH_2-CH_3$, sowie a = a1 = b1 = b2 = c = c1 = c2 = d = 0;
a2 = 2, b = 20 bis 400, b1 = 1 bis 20, $R^1 = CH_3$, $R^2$ = C6 bis C24 n-Alkyl, $R^3 = (CH_2)_3$- $O-CH_2-C(CH_2-O-C(O)-C(CH_3)=CH_2)_2-CH_2-CH_3$, sowie a = a1 = b2 = c = c1 = c2 = d = 0; oder
a + a2 =4, b = 20 bis 400, b1 = 1 bis 20, c = 2, $R^1 = R^5 = CH_3$, $R^2$ = C6 bis C24 n-Alkyl, $R^3 = (CH_2)_3-O-CH_2-C(CH_2-O-C(O)-C(CH_3)=CH_2)_2-CH_2-CH_3$, sowie a1 = b2 = c1 = c2 = d = 0.

9. Verfahren gemäß mindestens einem der vorgenannten Ansprüche, **dadurch gekennzeichnet, dass** das kosmetisch geeignete Trägermedium ausgewählt ist aus kosmetischen Ölen.

10. Verfahren gemäß mindestens einem der vorgenannten Ansprüche, **dadurch gekennzeichnet, dass** der organische Radikalstarter ein Peroxid ausgewählt ist aus der Gruppe der Peroxide, die sich von organischen Säuren, insbesondere Fettsäuren, ableiten, bevorzugt Dilauroylperoxid, Didecanoylperoxid und Diisononanoylperoxid, besonders bevorzugt Lauroyl-Peroxid.

11. Silikongel erhältlich nach dem Verfahren gemäß mindestens einem der Ansprüche 1 bis 10.

12. Silikongel gemäß Anspruch 11, **dadurch gekennzeichnet, dass** es bei einer Scherrate von 1 1/s und einer Temperatur von 25 °C eine Viskosität bestimmt nach der Methode in der Beschreibung von kleiner 150000 mPas, bevorzugt kleiner 100000 mPas, besonders bevorzug kleiner 50000 mPas insbesondere bevorzugt kleiner 15000 mPas, aufweist.

13. Kosmetische Formulierung enthaltend mindestens ein Silikongel gemäß Anspruch 11 oder 12.

14. Kosmetische Formulierung gemäß Anspruch 13, **dadurch gekennzeichnet, dass** sie 1 Gew.-% bis 40 Gew.-%,

bevorzugt 2 Gew.-% bis 30 Gew.-%, besonders bevorzugt 3 Gew.-% bis 24 Gew.-%, zur Herstellung der Formulierung eingesetzte Menge an Silikongel enthält.

**15.** Verwendung mindestens eines Silikongels gemäß Anspruch 11 oder 12 oder mindestens einer kosmetischen Formulierung gemäß Anspruch 13 oder 14 zur Konditionierung von Haut.

**Claims**

**1.** Process for the preparation of silicone gels comprising the process steps

A) provision of a mixture comprising
a silicone comprising
at least one (meth)acrylate group of the formula (I)

$$-O-C(O)-CR=CH_2 \qquad \text{formula (I)}$$

where R = -H or $-CH_3$,
with the proviso that the average molar ratio of (meth)acrylate groups to Si atoms in the silicone is in a range from 0.014 - 0.15, and
at least one cosmetically suitable carrier medium,
B) initiation of a free-radical polymerization by addition of at least one organic radical starter, preferably of an organic peroxide.

**2.** Process according to Claim 1, **characterized in that** the silicone used has at least one alkyl side chain having 2 to 30, preferably 8 to 24, particularly preferably 12 to 18, carbon atoms.

**3.** Process according to Claim 1 or 2, **characterized in that** the concentration of the silicone in the silicone/oil mixture is from 1 to 40% by weight, preferably from 3 to 30% by weight, particularly preferably from 5 to 25% by weight, based on the total weight of the silicone/oil mixture.

**4.** Process according to at least one of the preceding claims, **characterized in that** the average molar ratio of (meth)acrylate groups to Si atoms in the silicone is in the range from 0.016 to 0.12, particularly preferably from 0.018 to 0.10, especially preferably from 0.020 to 0.075.

**5.** Process according to at least one of the preceding claims, **characterized in that** the silicone has branched or unbranched alkyl radicals which are derived from long-chain fatty acids, in particular cetyl.

**6.** Process according to at least one of the preceding claims, **characterized in that** the silicone corresponds to one of the general formulae (II)

$$M_a\, M'_{a1}\, M''_{a2}\, D_b\, D'_{b1}\, D''_{b2}\, T_c\, T'_{c1}\, T''_{c2}\, Q_d \qquad \text{formula (II),}$$

where

$M = (R^1_3\ Si\ O_{1/2})$
$M' = (R^2R^1_2\ Si\ O_{1/2})$
$M'' = (R^3R^1_2\ Si\ O_{1/2})$
$D = (R^1_2\ Si\ O_{2/2})$
$D' = (R^2R^1\ Si\ O_{2/2})$
$D'' = (R^3R^1\ Si\ O_{2/2})$
$T = (R^5\ Si\ O_{3/2})$
$T' = (R^2\ Si\ O_{3/2})$
$T'' = (R^3\ Si\ O_{3/2})$
$Q = (Si\ O_{4/2})$
a = 0 to 32; preferably 0 to 22, in particular >= 2;
a1 = 0 to 32, preferably 0 to 22, in particular 0;
a2 = 0 to 32, preferably 0 to 22, in particular >= 2;

with the proviso that

$$a + a1 + a2 >= 2,$$

preferably = 2;

b = 1 to 600, preferably 10 to 500, in particular 20 to 400;
b1 = 0 to 80, preferably 0 to 50, in particular 1 to 20;
b2 = 0 to 50, preferably 0 to 20, in particular 0 to 10;
c = 0 to 30, preferably 0 to 20, in particular 0;
c1 = 0 to 10, preferably 0 to 5, in particular 0;
c2 = 0 to 10, preferably 0 to 5, in particular 0;
d = 0 to 15, preferably 0 to 12, in particular 0;

with the proviso that

$$a2 + b2 + c2 >= 1$$

and

$R^1$ = independently of one another identical or different linear or branched, optionally aromatic hydrocarbon radicals having 1 to 30 carbon atoms, preferably having 1 to 6 carbon atoms, particularly preferably methyl or phenyl, in particular methyl;
$R^2$ = independently of one another identical or different linear or branched, optionally aromatic hydrocarbon radicals having 2 to 30, preferably 7 to 22, carbon atoms, preferably linear saturated hydrocarbon radicals having 6 to 30, preferably 7 to 22 carbon atoms, particularly preferably linear saturated hydrocarbon radicals having 8 to 22, in particular 12 to 18 carbon atoms, in particular dodecyl, tetradecyl, hexadecyl and octadecyl radicals;
$R^3$ = independently of one another identical or different organic radicals which have a (meth)acrylate group of the formula (I)

-O-C(O)-CR=CH2 formula (I)

where R = -H or $-CH_3$;
$R^5$ = independently of one another identical or different radicals $R^1$, $R^2$ or $R^3$, preferably $R^1$, in particular methyl, phenyl, dodecyl or hexadecyl.

7. Process according to at least one of the preceding claims, **characterized in that** a silicone of the general formula (II) is used where

a + a1 + a2 = 2 and c + c1 + c2 + d = 0 or
a2 = 2 and c + c1 + c2 + d = 0.

8. Process according to at least one of the preceding claims, **characterized in that** a silicone of the general formula (II) is used where

a2 = 2, b = 20 to 400, b2 = 1 to 20, $R^1 = CH_3$, $R^3 = -C_6H_{12}-O-C(O)-C(CH_3)=CH_2$, and a = a1 = b1 = c = c1 = c2 = d = 0;
a2 = 2, b = 20 to 400, $R^1 = CH_3$, $R^3 = (CH_2)_3-O-CH_2-C(CH_2-O-C(O)-C(CH_3)=CH_2)_2-CH_2-CH_3$, and a = a1 = b1 = b2 = c = c1 = c2 = d = 0;
a2 = 2, b = 20 to 400, b1 = 1 to 20, $R^1 = CH_3$, $R^2$ = C6 to C24 n-alkyl, $R^3 = (CH_2)_3-O-CH_2-C(CH_2-O-C(O)-C(CH_3)=CH_2)_2-CH_2-CH_3$, and a = a1 = b2 = c = c1 = c2 = d = 0; or
a + a2 =4, b = 20 to 400, b1 = 1 to 20, c = 2, $R^1 = R^5 = CH_3$, $R^2$ = C6 to C24 n-alkyl, $R^3 = (CH_2)_3-O-CH_2-C(CH_2-O-C(O)-C(CH_3)=CH_2)_2-CH_2-CH_3$, and a1 = b2 = c1 = c2 = d = 0.

9. Process according to at least one of the preceding claims, **characterized in that** the cosmetically suitable carrier medium is selected from cosmetic oils.

10. Process according to at least one of the preceding claims, **characterized in that** the organic radical starter is a peroxide selected from the group of peroxides which are derived from organic acids, in particular fatty acids, preferably dilauroyl peroxide, didecanoyl peroxide and diisononanoyl peroxide, particularly preferably lauroyl peroxide.

11. Silicone gel obtainable by the process according to at least one of Claims 1 to 10.

12. Silicone gel according to Claim 11, **characterized in that**, at a shear rate of 1 1/s and a temperature of 25°C, it has a viscosity, determined according to the method in the description, of less than 150 000 mPas, preferably less than 100 000 mPas, particularly preferably less than 50 000 mPas, especially preferably less than 15 000 mPas.

13. Cosmetic formulation comprising at least one silicone gel according to Claim 11 or 12.

14. Cosmetic formulation according to Claim 13, **characterized in that** it comprises 1% by weight to 40% by weight, preferably 2% by weight to 30% by weight, particularly preferably 3% by weight to 24% by weight, amount of silicone gel used for preparing the formulation.

15. Use of at least one silicone gel according to Claim 11 or 12 or of at least one cosmetic formulation according to Claim 13 or 14 for the conditioning of skin.

## Revendications

1. Procédé de fabrication de gels de silicone comprenant les étapes de procédé suivantes :

A) la préparation d'un mélange comprenant :

une silicone contenant :

au moins un groupe (méth)acrylate de formule (I)

$$-O-C(O)-CR=CH_2 \qquad \text{Formule (I)}$$

avec R = -H ou $-CH_3$,
à condition que le rapport molaire moyen entre les groupes (méth)acrylate et les atomes de Si dans la silicone se situe dans une plage allant de 0,014 à 0,15, et
au moins un matériau support cosmétiquement approprié,

B) l'initiation d'une polymérisation radicalaire par ajout d'au moins un démarreur radicalaire organique, de préférence d'un peroxyde organique.

2. Procédé selon la revendication 1, **caractérisé en ce que** la silicone utilisée comprend au moins une chaîne latérale alkyle de 2 à 30, de préférence de 8 à 24, de manière particulièrement préférée de 12 à 18 atomes C.

3. Procédé selon la revendication 1 ou 2, **caractérisé en ce que** la concentration de la silicone dans le mélange silicone-huile est de 1 à 40 % en poids, de préférence de 3 à 30 % en poids, de manière particulièrement préférée de 5 à 25 % en poids, par rapport au poids total du mélange silicone-huile.

4. Procédé selon au moins l'une quelconque des revendications précédentes, **caractérisé en ce que** le rapport molaire moyen entre les groupes (méth)acrylate et les atomes Si dans la silicone se situe dans la plage allant de 0,016 à 0,12, de manière particulièrement préférée de 0,018 à 0,10, de manière notamment préférée de 0,020 à 0,075.

5. Procédé selon au moins l'une quelconque des revendications précédentes, **caractérisé en ce que** la silicone comprend des radicaux alkyle ramifiés ou non ramifiés, qui dérivent d'acides gras à chaîne longue, notamment cétyle.

**6.** Procédé selon au moins l'une quelconque des revendications précédentes, **caractérisé en ce que** la silicone correspond à une silicone de formule générale (II)

$$M_aM'_{a1}M''_{a2}D_bD'_{b1}D''_{b2}T_cT'c_1T''_{c2}Q_d \qquad \text{Formule (II)}$$

dans laquelle

$M = (R^1{}_3SiO_{1/2})$
$M' = (R^2R^1{}_2SiO_{1/2})$
$M'' = (R^3R^1{}_2SiO_{1/2})$
$D = (R^1{}_2SiO_{2/2})$
$D' = (R^2R^1SiO_{2/2})$
$D'' = (R^3R^1SiO_{2/2})$
$T = (R^5SiO_{3/2})$
$T' = (R^2SiO_{3/2})$
$T'' = (R^3SiO_{3/2})$
$Q = (SiO_{4/2})$
a = 0 à 32 ; de préférence 0 à 22, notamment ≥ 2 ;
a1 = 0 à 32 ; de préférence 0 à 22, notamment 0 ;
a2 = 0 à 32 ; de préférence 0 à 22, notamment ≥ 2 ;

à condition que

$$a + a1 + a2 \geq 2,$$

de préférence = 2 ;

b = 1 à 600, de préférence 10 à 500, notamment 20 à 400 ;
b1 = 0 à 80, de préférence 0 à 50, notamment 1 à 20 ;
b2 = 0 à 50, de préférence 0 à 20, notamment 0 à 10 ;
c = 0 à 30, de préférence 0 à 20, notamment 0 ;
c1 = 0 à 10, de préférence 0 à 5, notamment 0 ;
c2 = 0 à 10, de préférence 0 à 5, notamment 0 ;
d = 0 à 15, de préférence 0 à 12, notamment 0 ;

à condition que

$$a2 + b2 + c2 \geq 1$$

et

les $R^1$ = indépendamment les uns des autres, des radicaux hydrocarbonés identiques ou différents, linéaires ou ramifiés, éventuellement aromatiques, de 1 à 30 atomes de carbone, de préférence de 1 à 6 atomes de carbone, de manière particulièrement préférée méthyle ou phényle, notamment méthyle ;
les $R^2$ = indépendamment les uns des autres, des radicaux hydrocarbonés identiques ou différents, linéaires ou ramifiés, éventuellement aromatiques, de 2 à 30, de préférence de 7 à 22 atomes de carbone, de préférence des radicaux hydrocarbonés saturés linéaires de 6 à 30, de préférence 7 à 22 atomes de carbone, de manière particulièrement préférée des radicaux hydrocarbonés saturés linéaires de 8 à 22, notamment 12 à 18 atomes de carbone, notamment des radicaux dodécyle, tétradécyle, hexadécyle et octadécyle ;
les $R^3$ = indépendamment les uns des autres, des radicaux organiques identiques ou différents, qui comprennent un groupe (méth)acrylate de formule (I)

$$-O-C(O)-CR=CH_2 \qquad \text{Formule (I)}$$

avec R = -H ou $-CH_3$ ;
les $R^5$ - indépendamment les uns des autres, des radicaux $R^1$, $R^2$ ou $R^3$ identiques ou différents, de préférence $R^1$, notamment méthyle, phényle, dodécyle ou hexadécyle.

**7.** Procédé selon au moins l'une quelconque des revendications précédentes, **caractérisé en ce qu'**une silicone de formule générale (II) est utilisée, avec

$a + a1 + a2 = 2$ et $c + c1 + c2 + d = 0$, ou
$a2 = 2$ et $c + c1 + c2 + d = 0$.

**8.** Procédé selon au moins l'une quelconque des revendications précédentes, **caractérisé en ce qu'**une silicone de formule générale (II) est utilisée, avec

a2 = 2, b = 20 à 400, b2 = 1 à 20, $R^1 = CH_3$, $R^3 = -C_6H_{12}-O-C(O)-C(CH_3)=CH_2$, et a = a1 = b1 = c = c1 = c2 = d = 0 ;
a2 = 2, b = 20 à 400, $R^1 = CH_3$, $R^3 = (CH_2)_3-O-CH_2-C(CH_2-O-C(O)-C(CH_3)=CH_2)_2-CH_2-CH_3$, et a = a1 = b1 = b2 = c = c1 = c2 = d = 0 ;
a2 = 2, b = 20 à 400, b1 = 1 à 20, $R^1 = CH_3$, $R^2$ = n-alkyle en C6 à C24, $R^3 = (CH_2)_3-O-CH_2-C(CH_2-O-C(O)-C(CH_3)=CH_2)_2 -CH_2-CH_3$, et a = a1 = b2 = c = c1 = c2 = d = 0 ; ou
a + a2 = 4, b = 20 à 400, b1 = 1 à 20, c = 2, $R^1 = R^5 = CH_3$, $R^2$ = n-alkyle en C6 à C24, $R^3 = (CH_2)_3-O-CH_2-C(CH_2-O-C(O)-C(CH_3)=CH_2)_2-CH_2-CH_3$, et a1 = b2 = c1 = c2 = d = 0 .

**9.** Procédé selon au moins l'une quelconque des revendications précédentes, **caractérisé en ce que** le matériau support cosmétiquement approprié est choisi parmi les huiles cosmétiques.

**10.** Procédé selon au moins l'une quelconque des revendications précédentes, **caractérisé en ce que** le démarreur radicalaire organique est un peroxyde choisi dans le groupe des peroxydes qui dérivent d'acides organiques, notamment d'acides gras, de préférence le peroxyde de dilauroyle, le peroxyde de didécanoyle et le peroxyde de diisononanoyle, de manière particulièrement préférée le peroxyde de lauroyle.

**11.** Gel de silicone pouvant être obtenu par le procédé selon au moins l'une quelconque des revendications 1 à 10.

**12.** Gel de silicone selon la revendication 11, **caractérisé en ce qu'**il présente à un taux de cisaillement de 1 1/s et à une température de 25 °C une viscosité déterminée par la méthode indiquée dans la description inférieure à 150 000 mPas, de préférence inférieure à 100 000 mPas, de manière particulièrement préférée inférieure à 50 000 mPas, de manière notamment préférée inférieure à 15 000 mPas.

**13.** Formulation cosmétique contenant au moins un gel de silicone selon la revendication 11 ou 12.

**14.** Formulation cosmétique selon la revendication 13, **caractérisée en ce qu'**elle contient 1 % en poids à 40 % en poids, de préférence 2 % en poids à 30 % en poids, de manière particulièrement préférée 3 % en poids à 24 % en poids, quantité de gel de silicone utilisée pour la fabrication de la formulation.

**15.** Utilisation d'au moins un gel de silicone selon la revendication 11 ou 12 ou d'au moins une formulation cosmétique selon la revendication 13 ou 14 pour le conditionnement de la peau.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5760116 A
- US 6936686 B
- WO 12065822 A
- US 20030049212 A
- US 2004228821 A
- WO 2011049896 A
- EP 2273966 A1
- WO 2007115872 A


### In der Beschreibung aufgeführte Nicht-Patentliteratur


- **B. TIEKE.** Makromolekulare Chemie: Eine Beinführung. Wiley-VCH Verlag, 2005
- **A. YA. MALKIN.** *Rheokinetics: Rheological Transformations in Synthesis and Reactions of Oligomers and Polymers,* 1996
- **K. SCHRADER.** Grundlagen und Rezepturen der Kosmetika. Hüthig Buch Verlag Heidelberg, vol. 2, 329-341